# EUROPEAN PATENT APPLICATION

(11) **EP 4 592 274 A1**
(43) Date of publication of application: **30.07.2025**
(21) Application number: 23868216.5
(22) Date of filing: 20.09.2023
(51) Int. Cl.: C07C 229/34, A61K 41/00, A61K 51/04, A61P 35/00, A61P 35/02, C07B 59/00, C07F 5/02

(54) **RADIOLABELED TYROSINE DERIVATIVE AND APPLICATION THEREOF**

(30) Priority: 21.09.2022 JP 2022150608
(71) Applicant: OSAKA UNIVERSITY, Suita-shi Osaka 565-0871 (JP)
(72) Inventor: KANEDA, Kazuko, Suita-shi, Osaka 565-0871 (JP); SHIRAKAMI, Yoshifumi, Suita-shi, Osaka 565-0871 (JP); KADONAGA, Yuichiro, Suita-shi, Osaka 565-0871 (JP); TOYOSHIMA, Atsushi, Suita-shi, Osaka 565-0871 (JP); FUKASE, Koichi, Suita-shi, Osaka 565-0871 (JP); NAGATA, Hiroshi, Inashiki-gun, Ibaraki 300-0326 (JP)
(74) Representative: Weickmann & Weickmann PartmbB
(86) International application number: PCT/JP2023/034114
(87) International publication number: WO 2024/063095

(57) **Abstract**

The present invention provides radiolabeled tyrosine derivatives that have excellent LAT1 selectivity, have higher retention at tumor or cancer sites, and have a level of clearance that does not cause side effects, and that can be produced stably with good purity by a safe method suitable for industrial production.

The present invention provides a radiolabeled compound represented by Formula (I) or a pharmaceutically acceptable salt thereof: 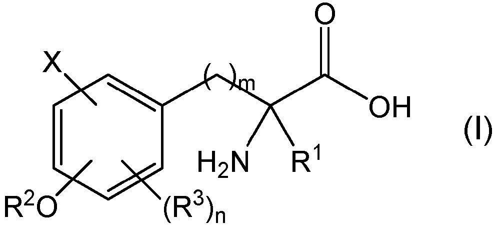 wherein each symbol is as defined in the present specification.

## Description

### [Technical Field]

The present invention relates to radiolabeled tyrosine derivatives useful as therapeutic agents for tumors or cancers, their use, methods for producing the tyrosine derivatives, and synthetic intermediates thereof.

### [Background Art]

Radiolabeled tyrosine derivatives, such as astato(²¹¹At)-α-methyl-L-tyrosine (hereinafter also referred to as ²¹¹At-AAMT), are drugs that are taken up into tumor cells via amino acid transporter LAT1 (L-type amino acid transporter 1, hereinafter also referred to as LAT1) which is expressed specifically in tumors, and are known to be useful as anti-cancer drugs (Patent Literature 1).

However, ²¹¹At-AAMT has a fast clearance, although its anti-tumor effect is observed. Therefore, the development of compounds with higher retention at tumor or cancer sites is desired. Furthermore, a major hurdle in drug development is the acquisition of drug resistance, which is believed to be caused by the overexpression of multidrug efflux transporters, such as ABC transporters. Compounds with high retention are expected to not only have stronger therapeutic effects at the same dose, but also to be applicable to the treatment of tumors or cancers that have acquired resistance to anti-cancer drugs.

Patent Literature 1 discloses that ²¹¹At-AAMT is conventionally produced by dissolving α-methyl-L-tyrosine (AMT) in sulfuric acid, adding mercury sulfate to the solution to introduce mercury on the benzene ring, and then subjecting the resulting compound to an astatine exchange reaction (hereinafter also referred to as mercury method). This method uses mercury, which is a hazardous substance. Therefore, it cannot be said to be a method suitable for the production of pharmaceuticals in terms of safety. In addition, the above method is not suitable for industrial production because the synthetic yield varies relatively greatly between production batches. Furthermore, iodine-substituted compounds and halogen-disubstituted compounds are produced as by-products, which causes problems in terms of purity.

On the other hand, the present inventors have already reported that a boryl group (-B(OH)₂) introduced into an aryl group has excellent astatine-substituting ability (Patent Literature 2).

### [Citation List]

### [Patent Literature]

[Patent Literature 1]
   WO 2019/176505
[Patent Literature 2]
   WO 2019/027059

### [Summary of Invention]

### [Technical Problem]

The present invention aims to provide radiolabeled tyrosine derivatives that have excellent LAT1 selectivity, have higher retention at tumor or cancer sites, and have a level of clearance that does not cause side effects, and that can be produced stably with good purity by a safe method suitable for industrial production.

### [Solution to Problem]

The present inventors have conducted intensive studies in an attempt to solve the above-mentioned problems and found that a novel compound represented by the following Formula (I) has excellent LAT1 selectivity, has higher retention at tumor or cancer sites, and has a level of clearance that does not cause side effects, and therefore can exhibit improved anti-tumor effects. In addition, the present inventors have also found that the above-mentioned compound can exhibit unexpectedly excellent anti-tumor effects even against tumors or cancers that have acquired resistance to anti-cancer drugs.

Furthermore, the present inventors have also found a method for producing a compound represented by the following Formula (I) without using hazardous substances such as mercury, via a new intermediate, a compound represented by the following Formula (II) and a compound represented by the following Formula (III), both into which a boryl group (-B(OH)₂) or its ester group is introduced.

As a result, the present invention has been completed.

Accordingly, the present invention provides the following.
[1] A radiolabeled compound represented by Formula (I) or a pharmaceutically acceptable salt thereof (hereinafter also referred to as Radiolabeled Compound (I)): 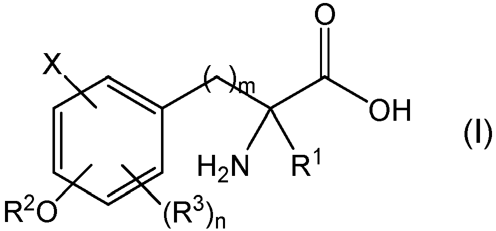 wherein
   R¹ is a C₁₋₄ alkyl group;
   R² is a C₁₋₆ alkyl group, a C₁₋₆ haloalkyl group or a C₇₋₁₄ aralkyl group;
   R³ is a fluorine atom or a chlorine atom;
   m is 0, 1 or 2;
   n is 0, 1 or 2; and
   X is a radionuclide selected from ²¹¹At, ²¹⁰At, ¹³¹I, ¹²⁵I, and ⁷⁷Br.
[2] The compound or a pharmaceutically acceptable salt thereof of the aforementioned [1], wherein m is 1.
[3] The compound or a pharmaceutically acceptable salt thereof of the aforementioned [1], wherein n is 0.
[4] The compound or a pharmaceutically acceptable salt thereof of the aforementioned [1], wherein R² is a C₁₋₆ alkyl group.
[5] The compound or a pharmaceutically acceptable salt thereof of the aforementioned [1], wherein R¹ is a methyl group.
[6] The compound or a pharmaceutically acceptable salt thereof of the aforementioned [1], wherein R²O is bonded to the 4-position on the benzene ring.
[7] The compound or a pharmaceutically acceptable salt thereof of the aforementioned [1], wherein X is bonded to the 3-position on the benzene ring.
[8] The compound or a pharmaceutically acceptable salt thereof of the aforementioned [1], wherein X is ²¹¹At.
[9] A pharmaceutical composition comprising the compound or a pharmaceutically acceptable salt thereof of any one of the aforementioned [1]-[8], and a pharmaceutically acceptable carrier.
[10] The pharmaceutical composition of the aforementioned [9], further comprising at least selected from the group consisting of ascorbic acid, an alkali metal ascorbate and an alkaline-earth metal ascorbate.
[11] A therapeutic agent for a tumor or cancer expressing amino acid transporter LAT1, comprising the compound or a pharmaceutically acceptable salt thereof of any one of the aforementioned [1]-[8].
[12] The therapeutic agent of the aforementioned [11], wherein the tumor or cancer expressing amino acid transporter LAT1 is selected from the group consisting of pancreatic cancer, leukemia, melanoma, colon cancer, lung cancer, prostate cancer, stomach cancer, breast cancer, kidney cancer, laryngeal cancer, esophageal cancer, liver cancer, lymphoma, myeloma, head and neck cancer, ovarian cancer, bladder cancer, childhood cancer, childhood leukemia, brain tumor, osteosarcoma, soft tissue sarcoma, and soft tissue tumor.
[13] The therapeutic agent of the aforementioned [11], wherein the tumor or cancer is a tumor or cancer that has acquired resistance to an anti-cancer drug.
[14] The therapeutic agent of the aforementioned [11], wherein the tumor or cancer is a tumor or cancer that has acquired resistance to a platinum preparation.
[15] The therapeutic agent of the aforementioned [11], wherein the tumor or cancer is a tumor or cancer that has acquired resistance to Cisplatin.
[16] The therapeutic agent of the aforementioned [11], wherein the tumor or cancer is a tumor or cancer that has acquired resistance to a nucleic acid synthesis inhibitor.
[17] The therapeutic agent of the aforementioned [11], wherein the tumor or cancer is a tumor or cancer that has acquired resistance to Gemcitabine.
[18] A compound represented by Formula (II) or a salt thereof (hereinafter also referred to as Boronic Acid Compound (II)): 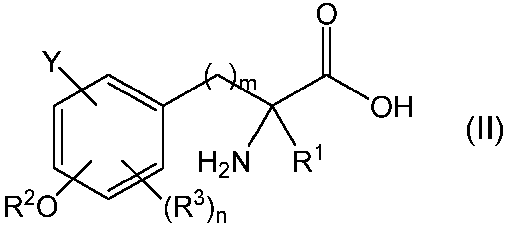 wherein
   R¹ is a C₁₋₄ alkyl group;
   R² is a C₁₋₆ alkyl group, a C₁₋₆ haloalkyl group or a C₇₋₁₄ aralkyl group;
   R³ is a fluorine atom or a chlorine atom;
   m is 0, 1 or 2;
   n is 0, 1 or 2; and
   Y is a boryl group (-B(OH)₂) or its ester group.
[19] The compound or a salt thereof of the aforementioned [18], wherein Y is a boryl group (-B(OH)₂) or a 4,4,5,5-tetramethyl-1,3,2-dioxaboran-2-yl group.
[20] An agent for boron neutron capture therapy, comprising the compound or a pharmaceutically acceptable salt thereof of the aforementioned [18] or [19].
[21] A method for producing a radiolabeled compound represented by Formula (I) or a pharmaceutically acceptable salt thereof, comprising the following step; 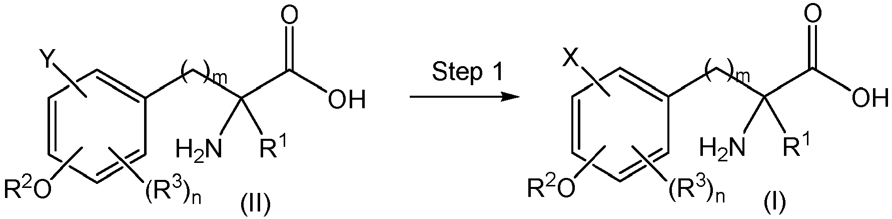 wherein
   R¹ is a C₁₋₄ alkyl group;
   R² is a C₁₋₆ alkyl group, a C₁₋₆ haloalkyl group or a C₇₋₁₄ aralkyl group;
   R³ is a fluorine atom or a chlorine atom;
   m is 0, 1 or 2;
   n is 0, 1 or 2;
   X is a radionuclide selected from ²¹¹At, ²¹⁰At, ¹³¹I, ¹²⁵I, and ⁷⁷Br; and
   Y is a boryl group (-B(OH)₂) or its ester group,
   Step 1: a step of reacting a compound represented by Formula (II) or a salt thereof with a radionuclide selected from ²¹¹At, ²¹⁰At, ¹³¹I, ¹²⁵I, and ⁷⁷Br in the presence of a reagent selected from alkali metal iodide, alkali metal bromide, N-bromosuccinimide, N-chlorosuccinimide, N-iodosuccinimide and hydrogen peroxide, in water to obtain a radiolabeled compound represented by Formula (I) or a pharmaceutically acceptable salt thereof.
[22] A compound represented by Formula (III) (hereinafter also referred to as Boronic Acid Compound (III)): 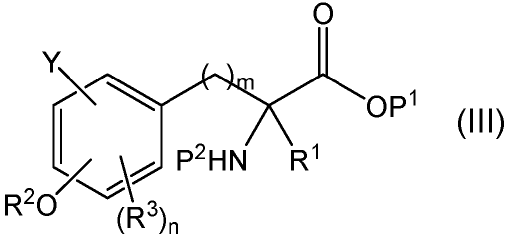 wherein
   R¹ is a C₁₋₄ alkyl group;
   R² is a C₁₋₆ alkyl group, a C₁₋₆ haloalkyl group or a C₇₋₁₄ aralkyl group;
   R³ is a fluorine atom or a chlorine atom;
   m is 0, 1 or 2;
   n is 0, 1 or 2;
   Y is a boryl group (-B(OH)₂) or its ester group;
   P¹ is a carboxy-protecting group; and
   P² is an amino-protecting group.
[23] The compound or a salt thereof of the aforementioned [22], wherein P¹ is a benzyl group, and P² is a tert-butoxycarbonyl group.
[24] A compound represented by Formula (IV'): 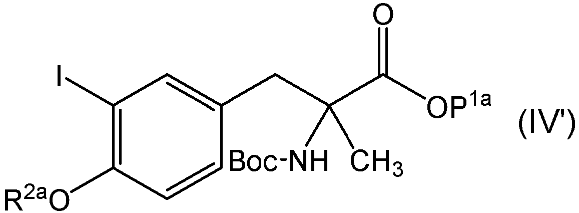 wherein R^{2a} is a C₁₋₃ alkyl group, and P^{1a} is a benzyl group or a C₁₋₃ alkyl group.
[24a] A compound represented by Formula (IV"): 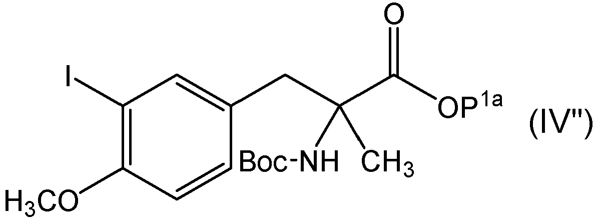 wherein P^{1a} is a benzyl group or a C₁₋₂ alkyl group.

### [Advantageous Effects of Invention]

According to the present invention, it is possible to provide compounds that have excellent LAT1 selectivity, have higher retention at tumor or cancer sites, and have a level of clearance that does not cause side effects, and therefore that can exhibit improved anti-tumor effects. It is also possible to provide compounds that can exhibit excellent anti-tumor effects even against tumors or cancers that have acquired resistance to anti-cancer drugs.

Furthermore, the compounds are produced via tyrosine derivatives (Boronic Acid Compound (III) and Boronic Acid Compound (II)), into which a boryl group (-B(OH)₂) or its ester group is introduced, and therefore, the compounds can be produced stably with good purity by a safe method suitable for industrial production of pharmaceuticals, without using any hazardous substances.

### [Brief Description of Drawings]

[Fig. 1]

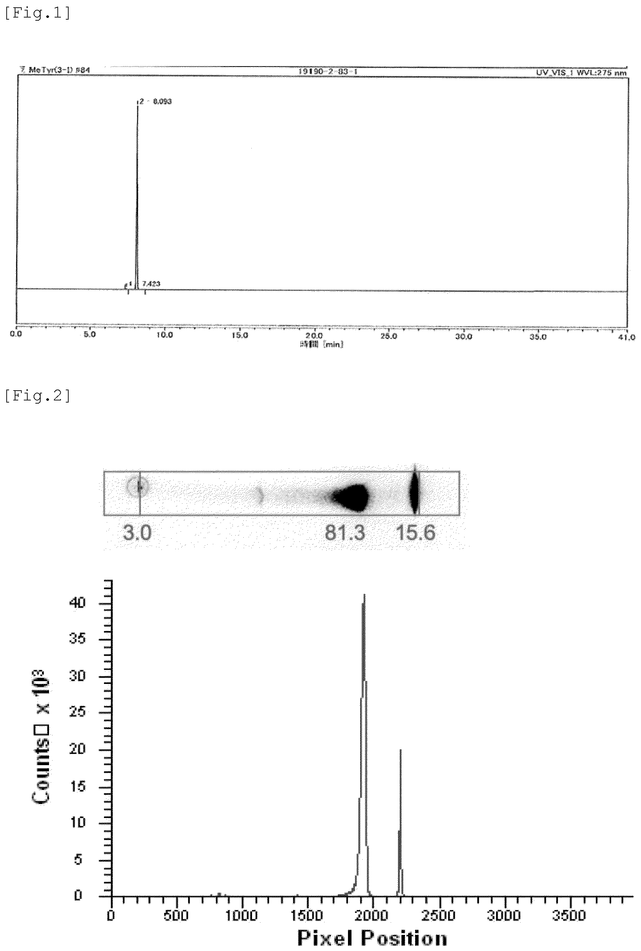

   Fig.1 is an HPLC chart of (S)-3-(4,4,5,5-tetramethyl-1,3,2-dioxaboran-2-yl)-O-methyl-α-methyltyrosine hydrochloride (6) (Bpin-AMT-OMe) obtained in Example 1.
[Fig. 2]
   Fig.2 shows the results of thin-layer chromatography (TLC) analysis of the crude product solution of ²¹¹At-AAMT-OMe obtained in Example 2.
[Fig. 3]

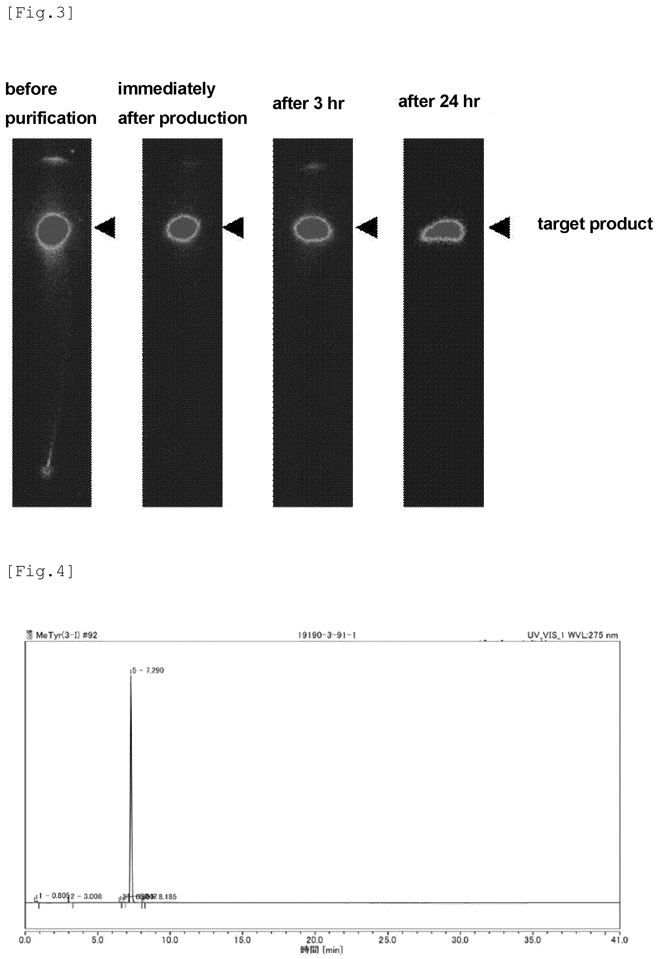

   Fig.3 shows the results of thin-layer chromatography (TLC) analysis of ²¹¹At-AAMT-OMe obtained in Example 2, performed before purification, immediately after production, 3 hours after production and 24 hours after production.
[Fig. 4]
   Fig.4 is an HPLC chart of (R)-3-(4,4,5,5-tetramethyl-1,3,2-dioxaboran-2-yl)-O-methyl-α-methyltyrosine hydrochloride (12) ((R)-Bpin-AMT-OMe) obtained in Example 3.
[Fig. 5]

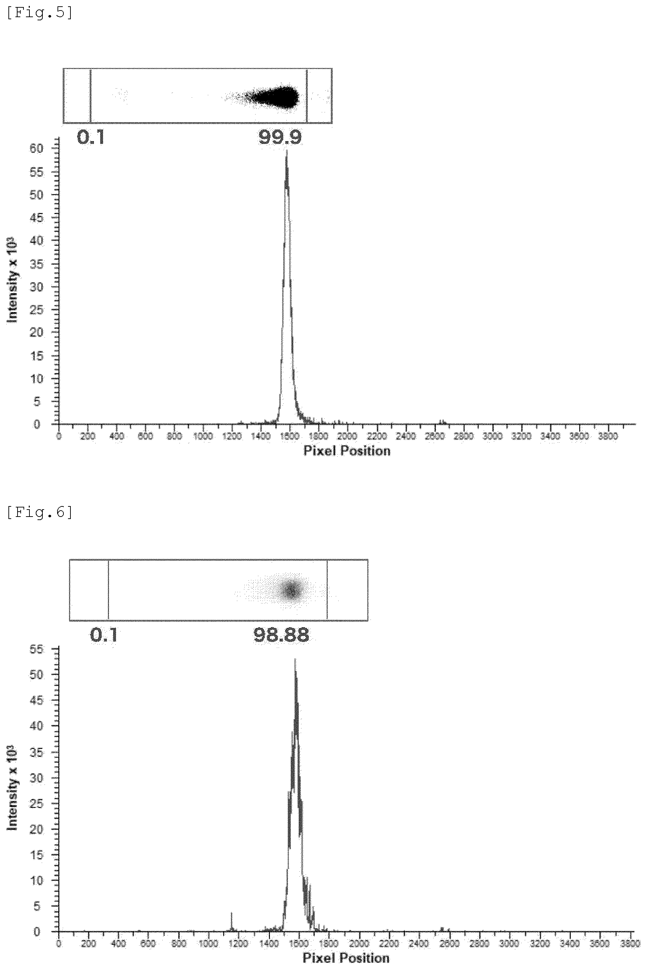

   Fig.5 shows the results of thin-layer chromatography (TLC) analysis of (R)-²¹¹At-AAMT-OMe obtained in Example 4, performed after purification.
[Fig. 6]
   Fig.6 shows the results of thin-layer chromatography (TLC) analysis of (R)-²²¹At-AAMT-OMe obtained in Example 4, performed 24 hours after production.
[Fig. 7]

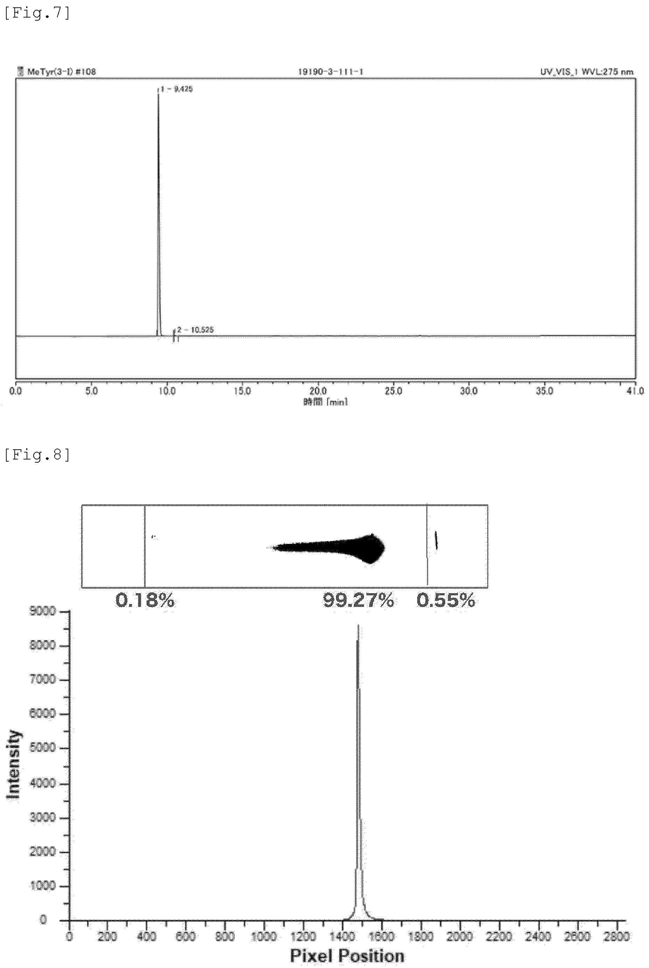

   Fig.7 is an HPLC chart of (S)-3-(4,4,5,5-tetramethyl-1,3,2-dioxaboran-2-yl)-O-ethyl-α-methyltyrosine hydrochloride (18) (Bpin-AMT-OEt) obtained in Example 5.
[Fig. 8]
   Fig.8 shows the results of thin-layer chromatography (TLC) analysis of ²¹¹At-AAMT-OEt obtained in Example 6, performed after purification.
[Fig. 9]

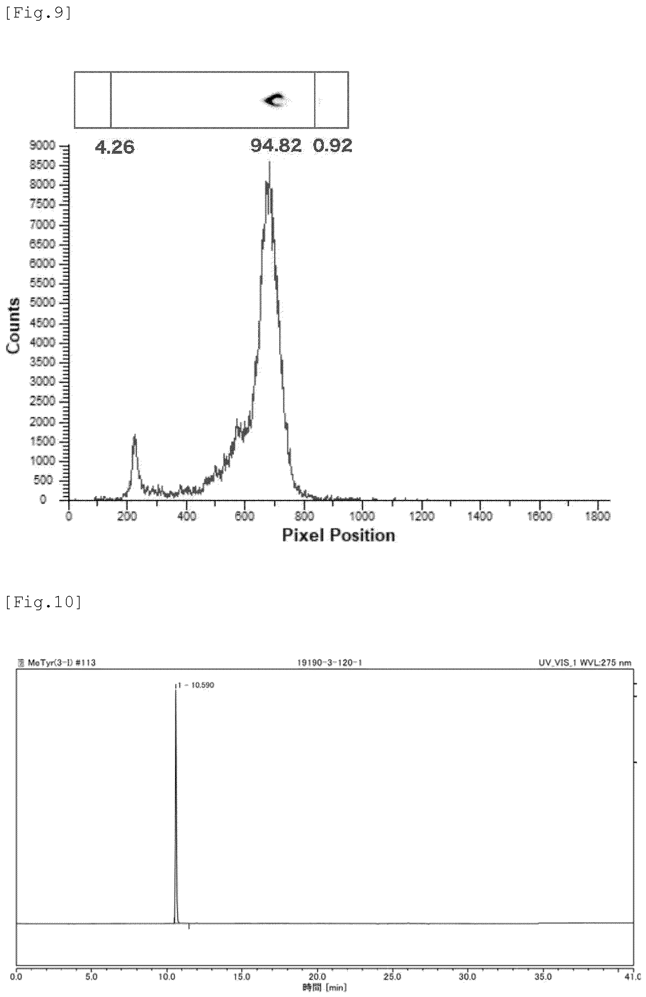

   Fig.9 shows the results of thin-layer chromatography (TLC) analysis of ²¹¹At-AAMT-OEt obtained in Example 6, performed 24 hours after production.
[Fig. 10]
   Fig.10 is an HPLC chart of (S)-3-(4,4,5,5-tetramethyl-1,3,2-dioxaboran-2-yl)-O-propyl-α-methyltyrosine hydrochloride (24) (Bpin-AMT-OPr) obtained in Example 7.
[Fig. 11]

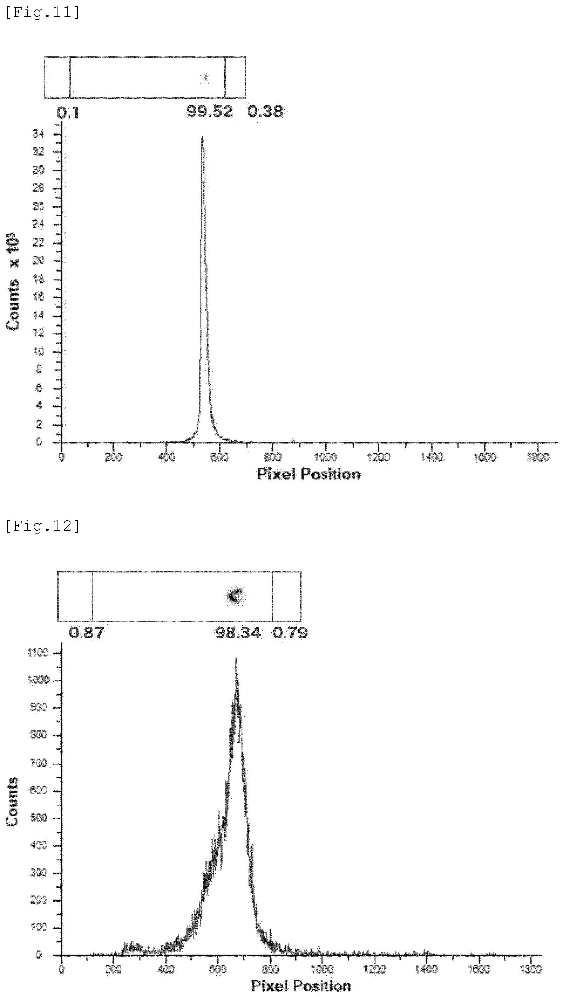

   Fig.11 shows the results of thin-layer chromatography (TLC) analysis of ²¹¹At-AAMT-OPr obtained in Example 8, performed after purification.
[Fig. 12]
   Fig.12 shows the results of thin-layer chromatography (TLC) analysis of ²¹¹At-AAMT-OPr obtained in Example 8, performed 24 hours after production.
[Fig. 13]

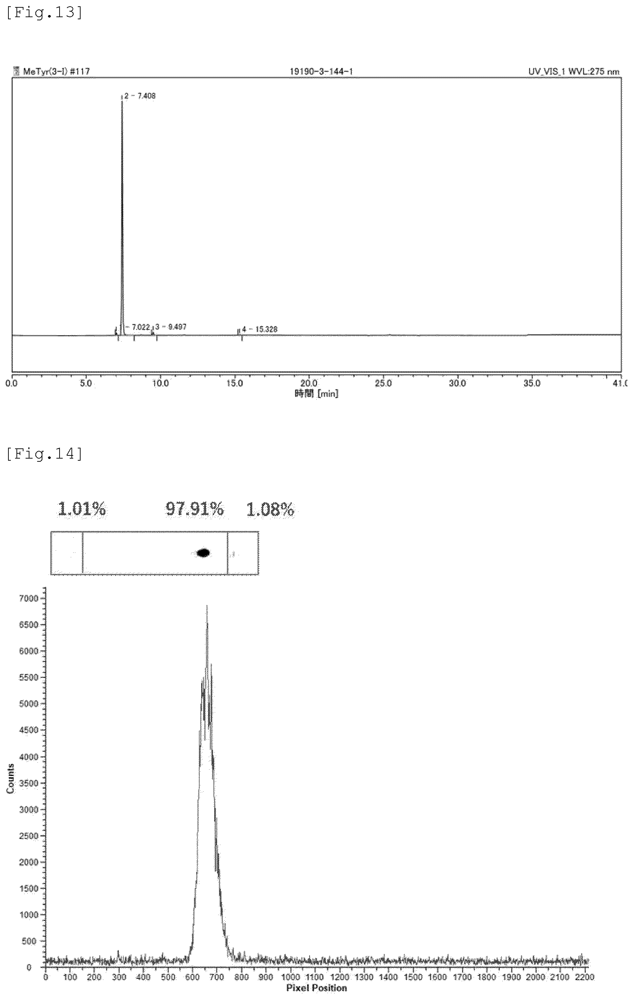

   Fig.13 is an HPLC chart of (S)-α-methyl-4-(4,4,5,5-tetramethyl-1,3,2-dioxaboran-2-yl)-3-methoxyphenylalanine hydrochloride (28) (Bpin-AMPhe-OMe) obtained in Example 9.
[Fig. 14]
   Fig.14 shows the results of thin-layer chromatography (TLC) analysis of ²¹¹At-AAMPhe-OMe obtained in Example 10, performed after purification.
[Fig. 15]

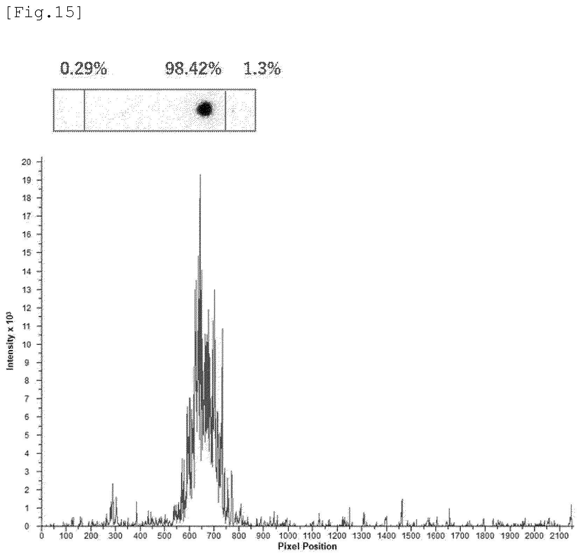

   Fig.15 shows the results of thin-layer chromatography (TLC) analysis of ²¹¹At-AAMPhe-OMe obtained in Example 10, performed 24 hours after production.
[Fig. 16]

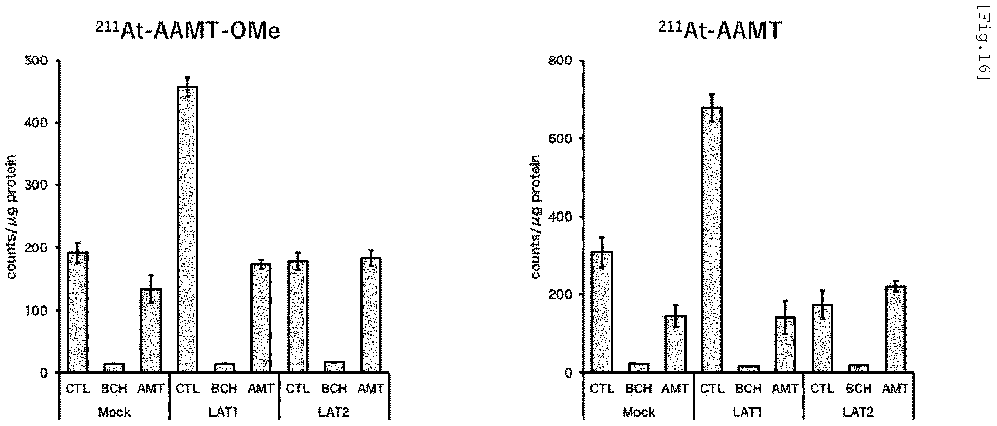

   Fig.16 shows the uptake level of ²¹¹At-AAMT-OMe or ²¹¹At-AAMT into cells (HEK293) in Test Example 1.
[Fig. 17]

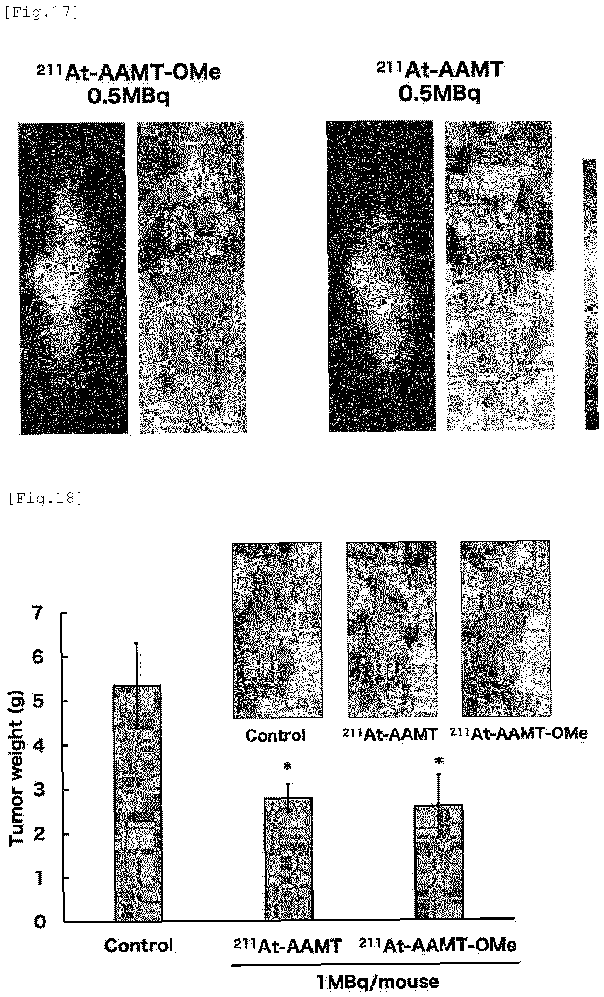

   Fig.17 shows the biodistribution of ²¹¹At-AAMT-OMe or ²¹¹At-AAMT 2 hours after administration to PANC1 subcutaneously transplanted mice in Test Example 2.
[Fig. 18]
   Fig.18 shows the tumor weight of PANC1 subcutaneously transplanted mice 28 days after administration of ²¹¹At-AAMT-OMe or ²¹¹At-AAMT in Test Example 3.
[Fig. 19]

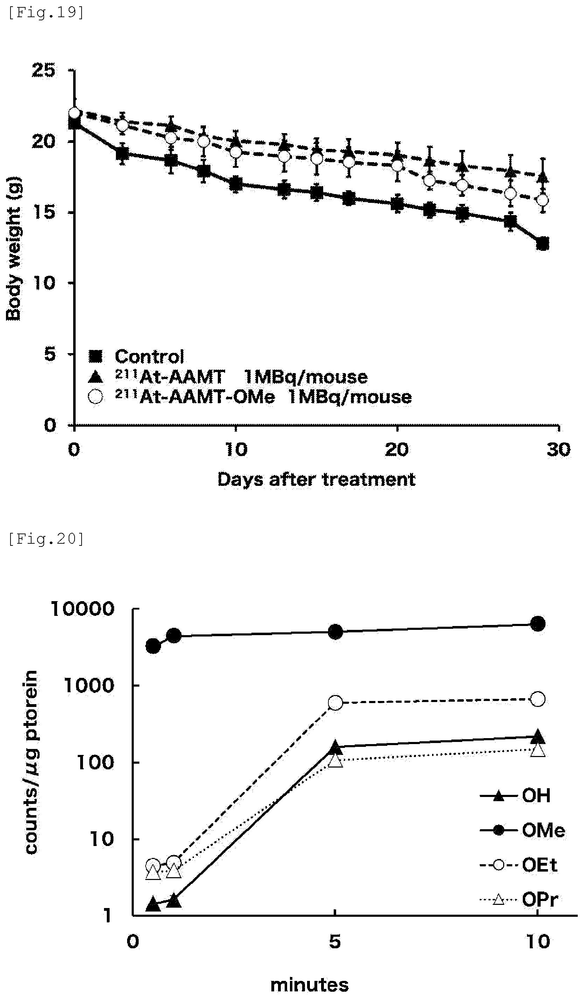

   Fig.19 shows the body weight of PANC1 subcutaneously transplanted mice from immediately after administration of ²¹¹At-AAMT-OMe or ²¹¹At-AAMT up to 28 days after administration in Test Example 3.
[Fig. 20]
   Fig.20 shows the uptake level of ²¹¹At-AAMT-OMe, ²¹¹At-AAMT-OEt, ²¹¹At-AAMT-OPr or ²¹¹At-AAMT into cell (PANC1) in Test Example 4.
[Fig. 21]

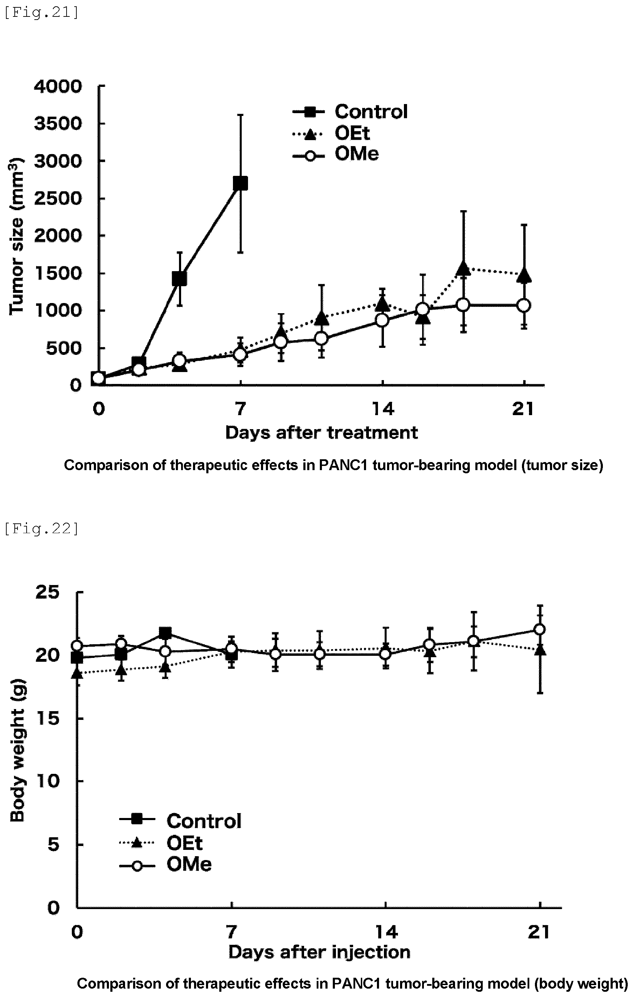

   Fig.21 shows the tumor size of PANC1 subcutaneously transplanted mice from immediately after administration of ²¹¹At-AAMT-OMe or ²¹¹At-AAMT-OEt up to 21 days after administration in Test Example 6.
[Fig. 22]
   Fig.22 shows the body weight of PANC1 subcutaneously transplanted mice from immediately after administration of ²¹¹At-AAMT-OMe or ²¹¹At-AAMT-OEt up to 21 days after administration in Test Example 6.
[Fig. 23]

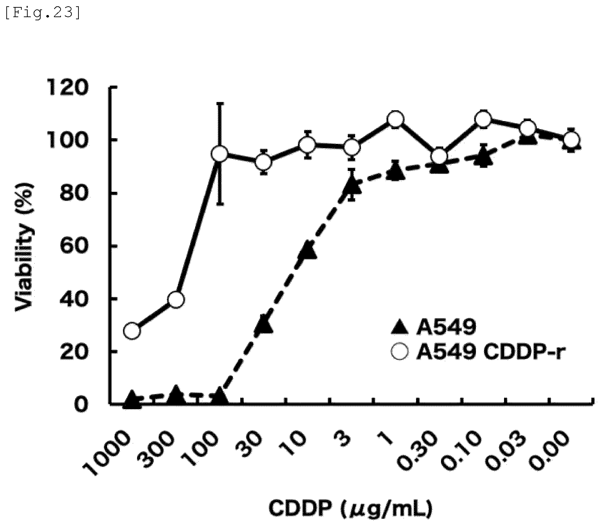

   Fig.23 shows the cell viability of A549 and CDDP-resistant A549 (A549 CDDP-r) in Test Example 7.
[Fig. 24]

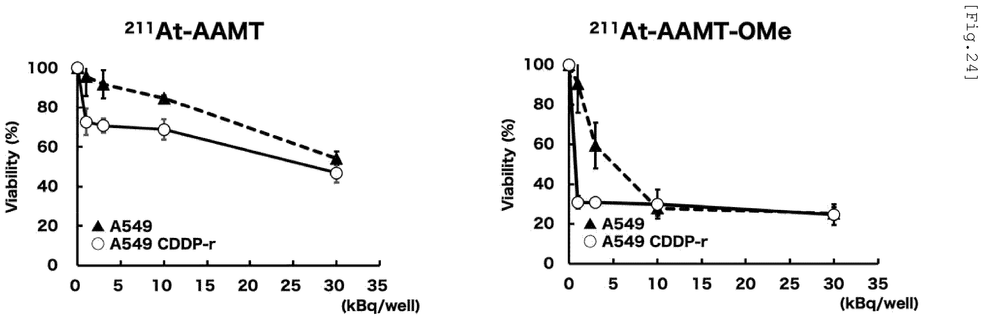

   Fig.24 shows the cell viability of A549 and CDDP-resistant A549 (A549 CDDP-r) after administration of ²¹¹At-AAMT-OMe or ²¹¹At-AAMT in Test Example 8.
[Fig. 25]

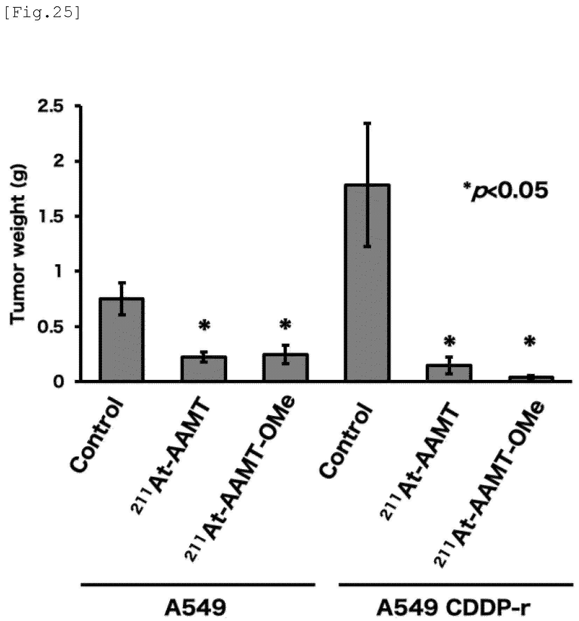

   Fig.25 shows the tumor weight of A549 transplanted mice and CDDP-resistant A549 (A549 CDDP-r) transplanted mice 30 days after administration of ²¹¹At-AAMT-OMe or ²¹¹At-AAMT in Test Example 9.
[Fig. 26]

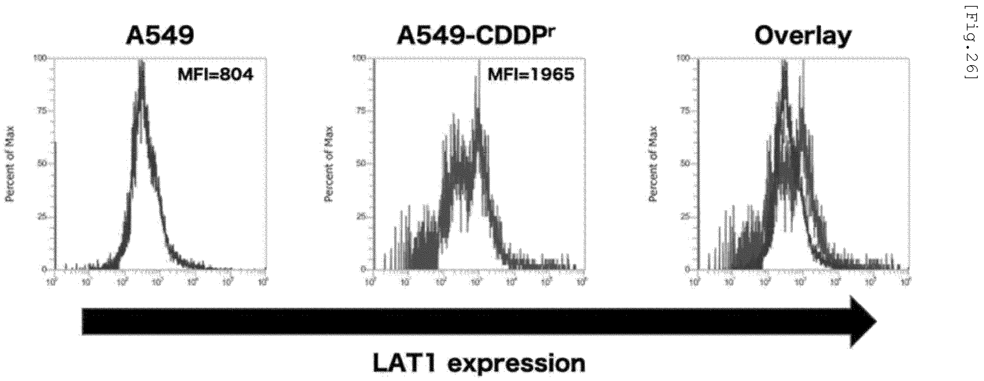

   Fig.26 shows the LAT1 expression level on the cell surface of A549 and CDDP-resistant A549 (A549 CDDP-r) in Test Example 10.
[Fig. 27]

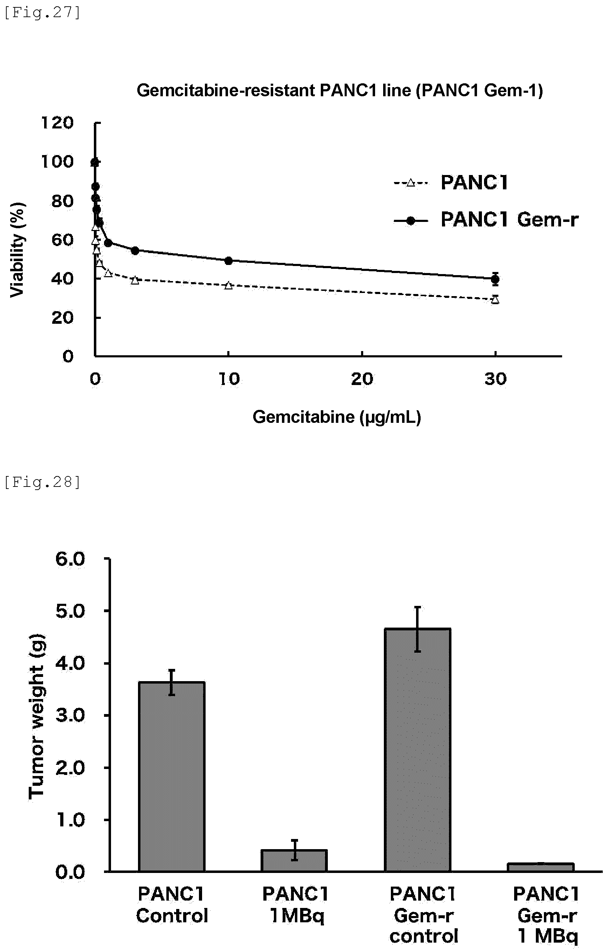

   Fig.27 shows the cell viability of PANC1 and Gemcitabine-resistant PANC1 (PANC1 Gem-r) in Test Example 11.
[Fig. 28]
   Fig.28 shows the tumor weight of PANC1 transplanted mice and Gemcitabine-resistant PANC1 (PANC1 Gem-r) transplanted mice 30 days after administration of ²¹¹At-AAMT-OMe in Test Example 12.
[Fig. 29]

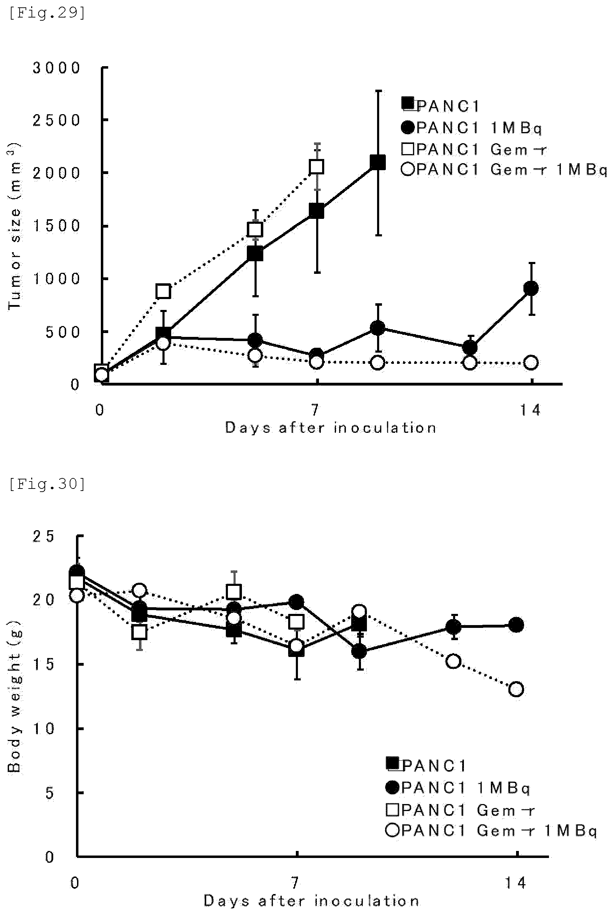

   Fig.29 shows the tumor size of PANC1 transplanted mice and Gemcitabine-resistant PANC1 (PANC1 Gem-r) transplanted mice from immediately after administration of ²¹¹At-AAMT-OMe up to 14 days after administration in Test Example 12.
[Fig. 30]
   Fig.30 shows the body weight of PANC1 transplanted mice and Gemcitabine-resistant PANC1 (PANC1 Gem-r) transplanted mice from immediately after administration of ²¹¹At-AAMT-OMe up to 14 days after administration in Test Example 12.

### [Description of Embodiments]

The present invention will be explained in detail below.

In the present specification, examples of the "halogen atom" include a fluorine atom, a chlorine atom, a bromine atom, and an iodine atom.

In the present specification, examples of the "C₁₋₄ alkyl group" include methyl, ethyl, propyl, isopropyl, butyl, isobutyl, sec-butyl and tert-butyl.

In the present specification, examples of the "C₁₋₆ alkyl group" include methyl, ethyl, propyl, isopropyl, butyl, isobutyl, sec-butyl, tert-butyl, pentyl, isopentyl, neo-pentyl, 1-ethylpropyl, hexyl, isohexyl, 1,1-dimethylbutyl, 2,2-dimethylbutyl, 3,3-dimethylbutyl, 2-ethylbutyl and the like.

In the present specification, the "C₁₋₆ haloalkyl group" means "C₁₋₆ alkyl group" substituted by one or more "halogen atom", and examples thereof include fluoromethyl, difluoromethyl, trifluoromethyl, chloromethyl, trichloromethyl, 2-fluoroethyl, 2-bromoethyl, 2,2,2-trifluoroethyl, pentafluoroethyl, 2,2-difluoropropyl, 3,3,3-trifluoropropyl, 4,4,4-trifluorobutyl, 5,5,5-trifluoropentyl, 6,6,6-trifluorohexyl and the like.

In the present specification, examples of the "C₆₋₁₀ aryl group" include phenyl, 1-naphthyl and 2-naphthyl.

In the present specification, the "C₇₋₁₄ aralkyl group" means "C₁₋₄ alkyl group" substituted by "C₆₋₁₀ aryl group", and examples thereof include benzyl, 1-phenethyl, 2-phenethyl, naphthyl methyl, phenylpropyl and the like.

In the present specification, the "boryl group (-B(OH)₂)" is also referred to as a dihydroxyboryl group.

In the present specification, examples of the "ester group of a boryl group" include the following groups. 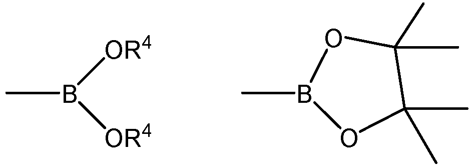 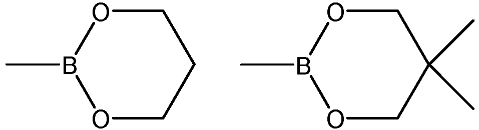 wherein R⁴ is a C₁₋₆ alkyl group.

In the present specification, examples of the "amino-protecting group" include a tert-butoxycarbonyloxy group, a benzyloxycarbonyl group, a 9-fluorenylmethyloxycarbonyl group and the like.

In the present specification, examples of the "carboxy-protecting group" include a benzyl group, a C₁₋₂ alkyl group (a methyl group, an ethyl group), a tert-butyl group and the like.

Radiolabeled Compound (I) of the present invention is the compound shown below.

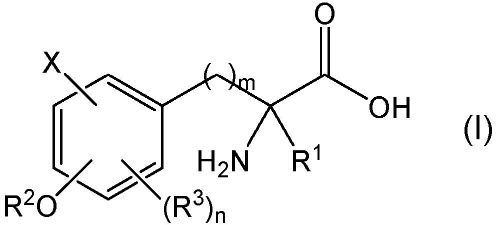

R¹ is a C₁₋₄ alkyl group.

In one embodiment, R¹ is preferably a methyl group.

R² is a C₁₋₆ alkyl group (preferably a methyl group, an ethyl group, a propyl group), a C₁₋₆ haloalkyl group (preferably a trifluoromethyl group, a 2-fluoroethyl group) or a C₇₋₁₄ aralkyl group (preferably a benzyl group).

In one embodiment, R² is preferably a methyl group, an ethyl group, a propyl group, a trifluoromethyl group, a 2-fluoroethyl group, or a C₇₋₁₄ aralkyl group, more preferably a methyl group, an ethyl group, a propyl group, or a trifluoromethyl group, still more preferably a methyl group, an ethyl group, or a propyl group, particularly preferably a methyl group.

In another embodiment, R² is preferably a C₁₋₆ alkyl group, more preferably a C₁₋₄ alkyl group, still more preferably a methyl group, an ethyl group, or a propyl group, particularly preferably a methyl group.

R³ is a fluorine atom or a chlorine atom.

In one embodiment, R³ is preferably a fluorine atom.

n is 0, 1 or 2.

In one embodiment, n is preferably 0.

m is 0, 1 or 2.

In one embodiment, m is preferably 1.

X is a radionuclide selected from ²¹¹At (α-ray emitting nuclide), ²¹⁰At (α-ray emitting nuclide), ¹³¹I (β-ray emitting nuclide), ¹²⁵I (X-ray emitting nuclide), and ⁷⁷Br (auger electron emitting nuclide).

The half-lives of these radionuclides are 7.2 hours for ²¹¹At, 8.3 hours for ²¹⁰At, 8.04 days for ¹³¹I, 59.4 days for ¹²⁵I, and 57 hours for ⁷⁷Br.

In one embodiment, X is preferably ²¹¹At.

The binding position of R²O on the benzene ring is not particularly limited, but is preferably the 3-position or the 4-position, particularly preferably the 4-position.

The binding position of X on the benzene ring is not particularly limited. For example, when R²O is bonded to the 4-position on the benzene ring, then the binding position of X is preferably the 3-position, and when R²O is bonded to the 3-position on the benzene ring, when the binding position of X is preferably the 4-position.

The binding position of R³ on the benzene ring is not particularly limited, but is preferably the 2-position, the 5-position or the 6-position, particularly preferably the 5-position.

The configuration of Formula (I) is not particularly limited and may be any of the D-, L-, or DL-form (i.e., any of the R-, S-, or R/S-form). That is, Radiolabeled Compound (I) may be any of Radiolabeled Compound (Ia) and Compound (Ib) represented by the following formulae, and racemates thereof. 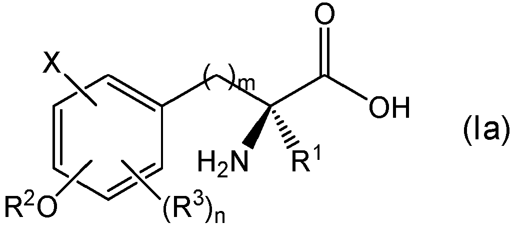 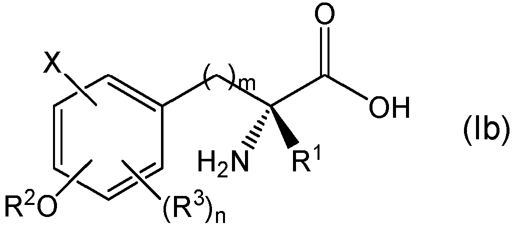 wherein each symbol is as defined above.

In one embodiment, in terms of uptake into tumor or cancer cells, Radiolabeled Compound (I) is preferably Radiolabeled Compound (Ia).

Specific examples of Radiolabeled Compound (I) include the following compounds. 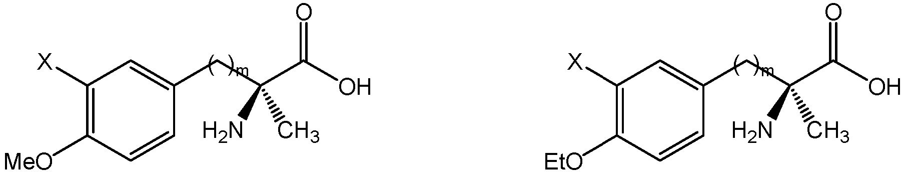 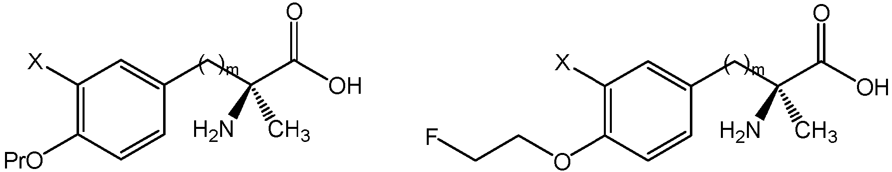 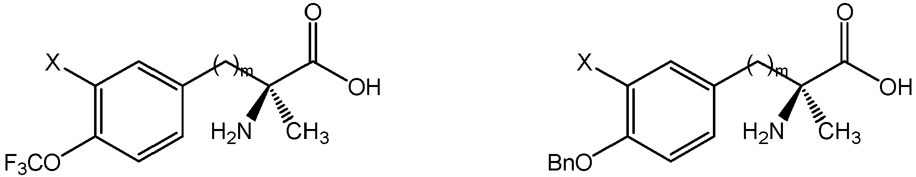 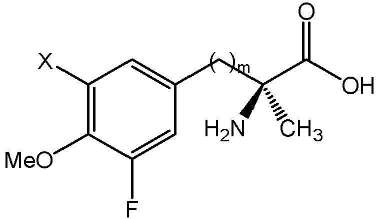 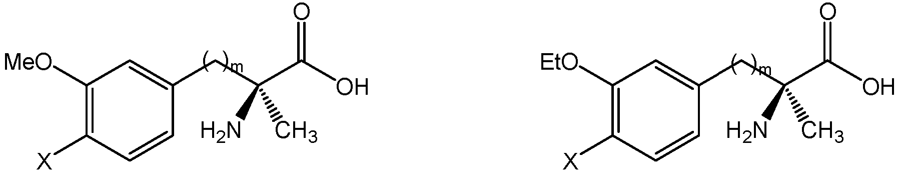 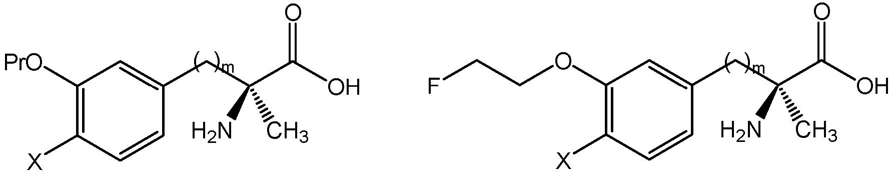 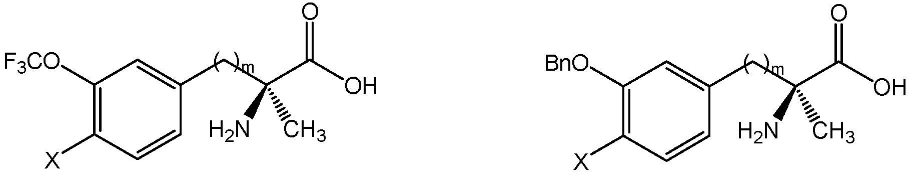 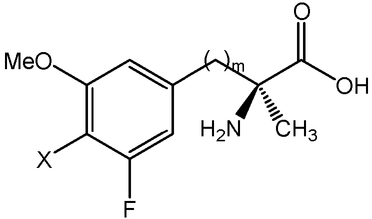 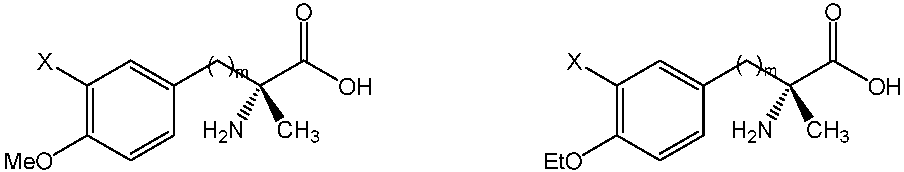 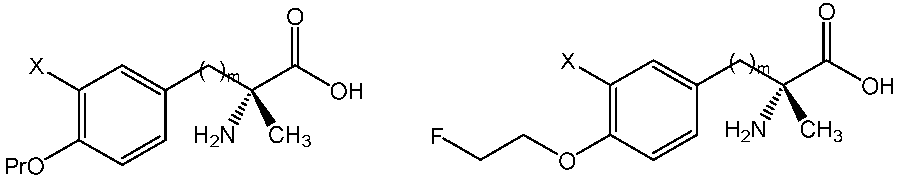 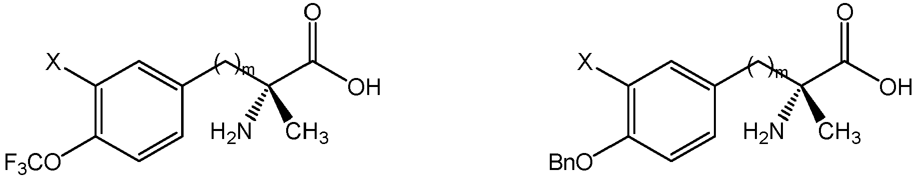 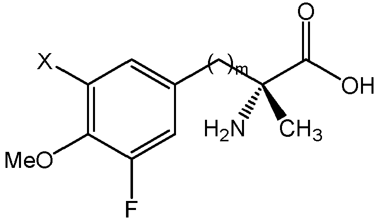 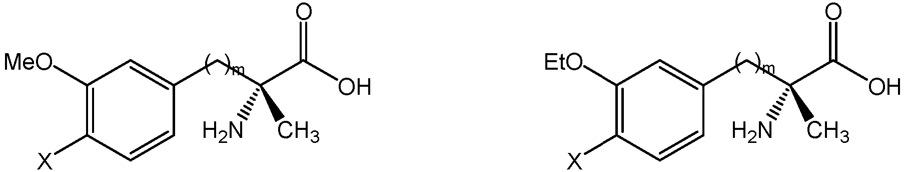 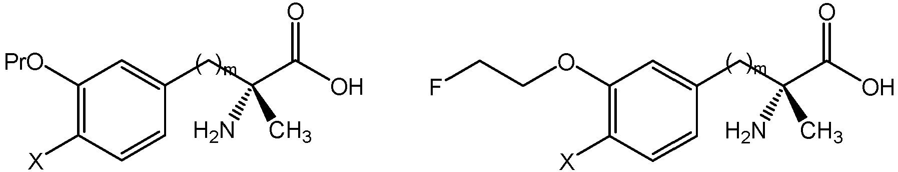 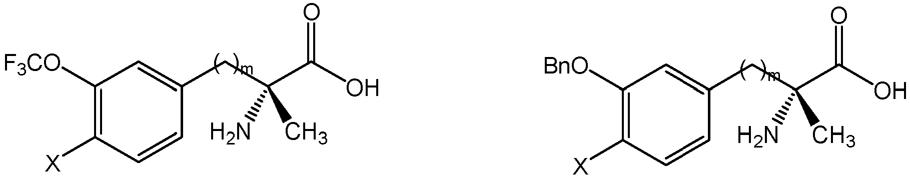 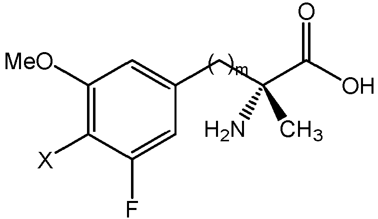 wherein Me is a methyl group, Et is an ethyl group, Pr is a propyl group, Bn is a benzyl group, X is as defined above, preferably ²¹¹At, and m is as defined above, preferably 1.

The compound represented by Formula (I) may be in the form of a pharmaceutically acceptable salts thereof. Examples of the pharmaceutically acceptable salt include inorganic salts such as alkali metal salts (e.g., sodium salt, potassium salt, etc.), alkaline-earth metal salts (e.g., calcium salt, magnesium salt, barium salt, etc.) and the like, ammonium salts and the like, salts with inorganic acids such as hydrogen chloride, hydrogen bromide, nitric acid, sulfuric acid, phosphoric acid and the like, and salts with organic acids such as acetic acid, phthalic acid, fumaric acid, oxalic acid, tartaric acid, maleic acid, citric acid, succinic acid, methanesulfonic acid, p-toluenesulfonic acid and the like.

The method for producing Radiolabeled Compound (I) of the present invention will be explained below.

In the present specification, when a raw material compound is in the form of a salt, examples of such salt include metal salts (e.g., alkali metal salts such as sodium salt, potassium salt and the like; alkaline-earth metal salts such as calcium salt, magnesium salt, barium salt and the like), ammonium salts, salts with organic bases (e.g., trimethylamine, triethylamine, pyridine, picoline, 2,6-lutidine), salts with inorganic acids (e.g., hydrogen chloride, hydrogen bromide, nitric acid, sulfuric acid, phosphoric acid), salts with organic acids (e.g., formic acid, acetic acid, trifluoroacetic acid, phthalic acid, fumaric acid, oxalic acid, tartaric acid, maleic acid, citric acid, succinic acid, malic acid, methanesulfonic acid, p-toluenesulfonic acid), and the like.

Radiolabeled Compound (I) can be produced by the method comprising the following Step 1. 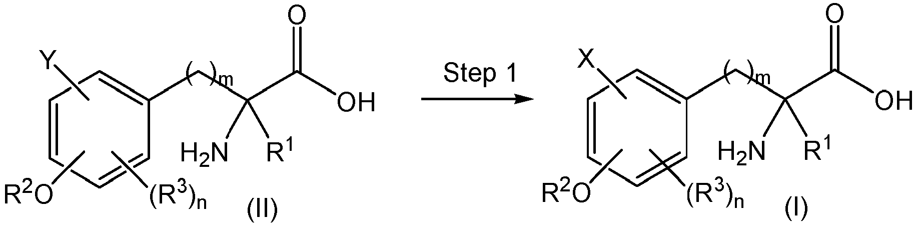 wherein Y is a boryl group (-B(OH)₂) or its ester group, and the other symbols are as defined above.

In one embodiment, Y is preferably a boryl group (-B(OH)₂) or a 4,4,5,5-tetramethyl-1,3,2-dioxaboran-2-yl group, particularly preferably a 4,4,5,5-tetramethyl-1,3,2-dioxaboran-2-yl group.

Step 1 is a step of reacting Boronic Acid Compound (II) with a radionuclide selected from ²¹¹At, ²¹⁰At, ¹³¹I, ¹²⁵I, and ⁷⁷Br, in the presence of a reagent selected from an alkali metal iodide, an alkali metal bromide, N-bromosuccinimide, N-chlorosuccinimide, N-iodosuccinimide and hydrogen peroxide, in water to obtain Radiolabeled Compound (I).

Boronic Acid Compound (II) is a novel compound.

Boronic Acid Compound (II) may be any of Boronic Acid Compound (IIa) and Compound (IIb) represented by the following formulae, and racemates thereof. 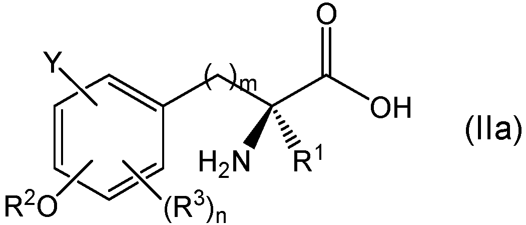 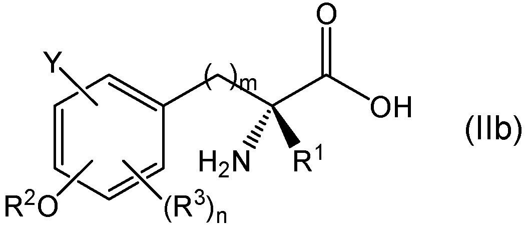 wherein each symbol is as defined above.

In one embodiment, Boronic Acid Compound (II) is preferably Boronic Acid Compound (IIa).

Specific examples of Boronic Acid Compound (II) include the following compounds. 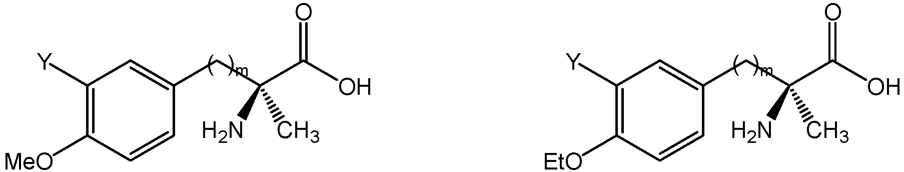 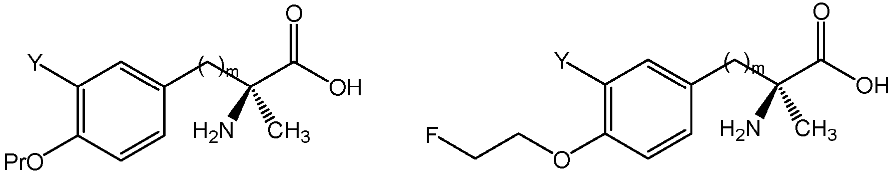 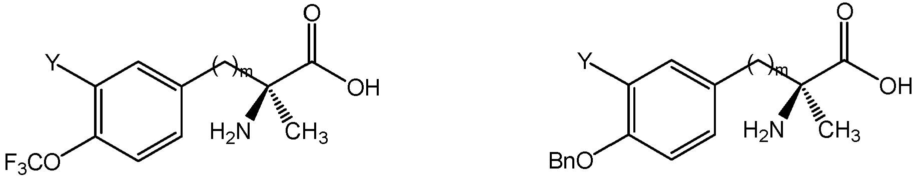 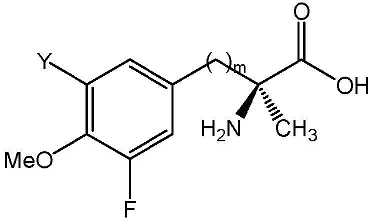 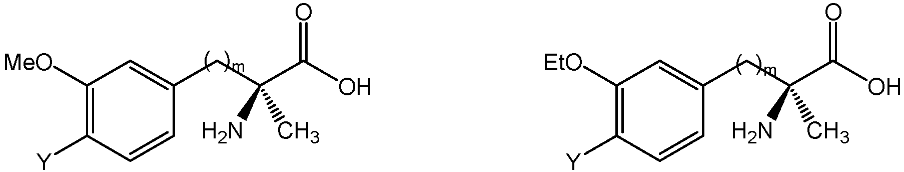 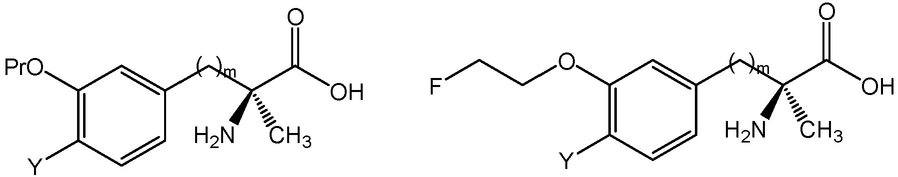 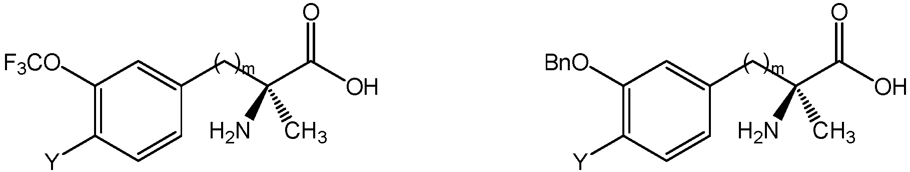 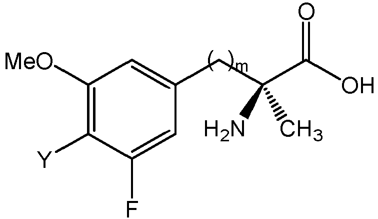 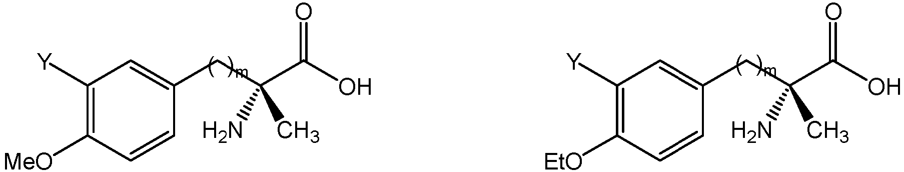 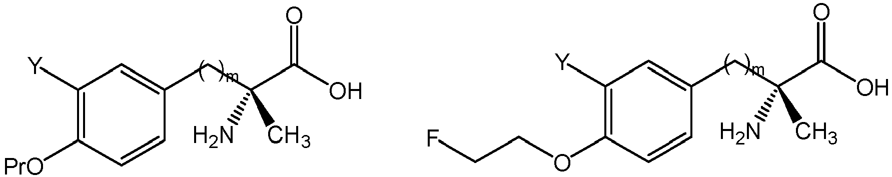 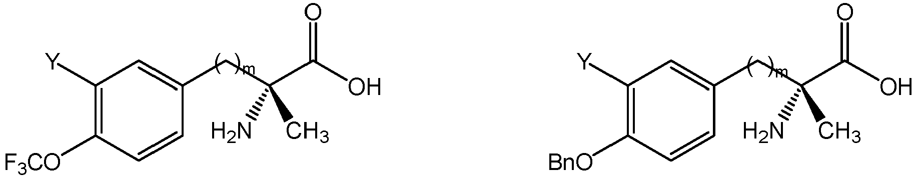 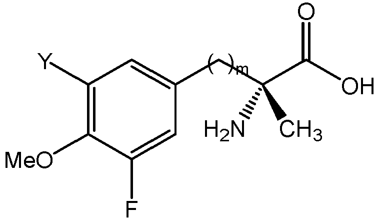 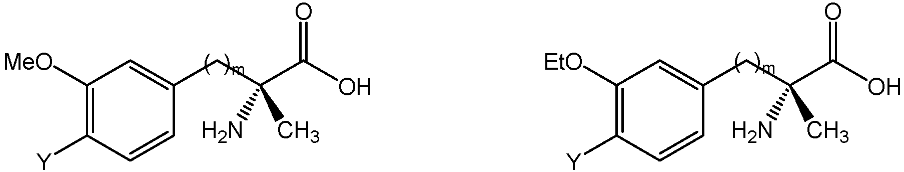 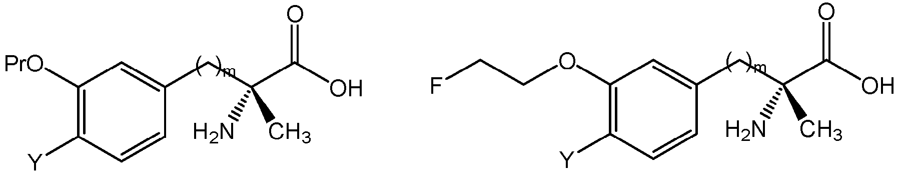 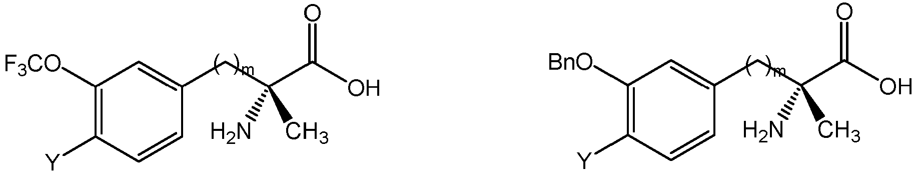 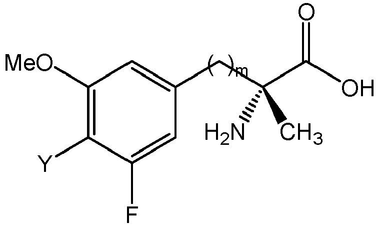 wherein Y is as defined above, preferably a boryl group (-B(OH)₂) or a 4,4,5,5-tetramethyl-1,3,2-dioxaboran-2-yl group, particularly preferably a 4,4,5,5-tetramethyl-1,3,2-dioxaboran-2-yl group, m is as defined above, preferably 1, and Me, Et, Pr and Bn are as defined above.

Boronic Acid Compound (II) can be produced by the method described below.

Since the reaction in this step is carried out in water, Boronic Acid Compound (II) may be in a free form or a salt form as long as it can be dissolved in water. Alternatively, it may be used in the form of a solution prepared by dissolving in a weakly basic aqueous solution such as an aqueous sodium hydrogen carbonate solution.

Examples of the alkali metal iodide include potassium iodide, sodium iodide and the like. Among them, potassium iodide is preferably used.

Examples of the alkali metal bromide include sodium bromide, potassium bromide and the like.

Preferred combinations of the radionuclide and the above reagent include
(1) a combination in which the radionuclide is ²¹¹At or ²¹⁰At, and the above reagent is selected from potassium iodide, sodium bromide, N-bromosuccinimide, N-chlorosuccinimide, N-iodosuccinimide and hydrogen peroxide;
(2) a combination in which the radionuclide is ¹²⁵I or ¹³¹I, and the above reagent is selected from N-bromosuccinimide and N-chlorosuccinimide; and
(3) a combination in which the radionuclide is ⁷⁷Br, and the above reagent is N-chlorosuccinimide.
The above reagent may be used alone or in combination of two or more. The above reagent is used usually in the form of an aqueous solution.

Preferred embodiments include a combination in which the radionuclide is ²¹¹At or ¹³¹I, and the above reagent is selected from potassium iodide and N-bromosuccinimide.

More preferred embodiments include
a combination in which the radionuclide is ²¹¹At, and the above reagent is potassium iodide, and
a combination in which the radionuclide is ¹³¹I, and the above reagent is N-bromosuccinimide.

The above reagent is used in an amount sufficient to oxidize or reduce the radionuclide, and is used usually in a large excess amount relative to the radionuclide. It is used preferably in a concentration of 0.0001 to 0.2 mol/L, more preferably in a concentration of 0.001 to 0.1 mol/L, in terms of reaction efficiency and economic efficiency.

For the reaction, the radionuclide is used usually in the form of an aqueous solution. If necessary, an alkaline aqueous solution such as sodium hydroxide and buffer solution may be added to the aqueous solution in order to stabilize the radionuclide.

In the case of radionuclide ²¹¹At, first, ²¹¹At is produced by ²⁰⁹Bi(α,2n)²¹¹At nuclear reaction resulting from the irradiation of bismuth with helium particles accelerated to 28 MeV by cyclotron. Next, by heating, the target substance ²⁰⁹Bi is melted and the ²¹¹At is vaporized, and the vaporized ²¹¹At is collected in a cooling trap, and dissolved in water to prepare an ²¹¹At stock solution. If necessary, an alkaline solution such as sodium hydroxide and buffer solution may be added thereto for the purpose of stabilizing ²¹¹At.

In the case of radionuclide ²¹⁰At, first, ²¹⁰At is produced by ²⁰⁹Bi(α,3n)²¹⁰At nuclear reaction resulting from the irradiation of bismuth with helium particles accelerated to 29 MeV or more by cyclotron. Next, by the same procedures as above, an aqueous ²¹⁰At solution is prepared.

In the case of radionuclide ¹²⁵I, it is available as an aqueous Na¹²⁵I solution.

In the case of radionuclide ¹³¹I, it is available as an aqueous Na¹³¹I solution.

In the case of radionuclide ⁷⁷Br, first, ⁷⁷Br is produced by ⁷⁷Se(p,n)⁷⁷Br nuclear reaction resulting from the irradiation of tellurium with proton particles accelerated by cyclotron. Next, the target substance ⁷⁷Se is melted, and the ⁷⁷Br is vaporized to prepare an aqueous ⁷⁶Br sodium hydroxide solution.

²¹¹At has a half-life of 7.2 hours, and ²¹⁰At has a half-life of 8.3 hours. These radionuclides have a short half-life, and therefore, they should be used in the subsequent reaction immediately after the preparation. On the other hand, ¹²⁵I has a half-life of 59.4 days, ¹³¹I has a half-life of 8.04 days, and ⁷⁷Br has a half-life of 57 hours. Although these radionuclides have a relatively long half-life, they are also preferably used in the subsequent reaction immediately after the preparation.

Boronic Acid Compound (II) is used usually in a large excess amount relative to the radionuclide, preferably in a concentration of 0.00001 mol/l to 0.5 mol/l, more preferably in a concentration of 0.0001 mol/l to 0.2 mol/l, per 1 Bq to 1,000 GBq of the radionuclide, in terms of reaction efficiency and economic efficiency.

The above reaction is carried out by mixing Boronic Acid Compound (II), the above reagent and the radionuclide, and the mixing order is not particularly limited. The reaction is preferably carried out by adding an aqueous solution of the radionuclide, followed by an aqueous solution of the above reagent to an aqueous solution of Boronic Acid Compound (II), or by adding an aqueous solution of the above reagent, followed by an aqueous solution of the radionuclide to an aqueous solution of Boronic Acid Compound (II), more preferably by adding an aqueous solution of the radionuclide, followed by an aqueous solution of the above reagent to an aqueous solution of Boronic Acid Compound (II).

The above reaction is carried out in water, i.e., in an organic solvent-free system.

The above reaction is carried out within the range of 0 to 95°C, preferably 10 to 80°C. The reaction time is from 1 minute to 3 hours, preferably from 1 minute to 1 hour.

The completion of the reaction is confirmed by the disappearance of the free radionuclide, using thin-layer chromatography (TLC) analysis.

The production method of the present invention makes it possible to obtain Radiolabeled Compound (I) in a high radiochemical yield (RCY) of 60% or more, particularly 80% or more, especially 90% or more.

Since the reaction solution after the completion of the reaction contains neither an organic solvent nor a toxic reagent, the reaction solution can be immediately formulated into an injection and the like without isolating Radiolabeled Compound (I).

The reaction of Boronic Acid Compound (II) with a radionuclide is an electrophilic substitution reaction and/or nucleophilic substitution reaction. The introduction site of the radionuclide in Boronic Acid Compound (II) is the benzene ring, and especially in the case of ²¹¹At or ²¹⁰At, the radionuclide is well introduced into the benzene ring.

Radiolabeled Compound (I) may be purified to remove by-products, if necessary. This purification is preferably carried out by a solid-phase extraction column. As solid-phase extraction columns, those commonly used in the technical field can be used.

Furthermore, after the above purification, ascorbic acid, or an alkali metal ascorbate or an alkaline-earth metal ascorbate may be added to a final concentration of 0.01% to 10%, preferably 0.1% to 5%. This makes it possible to suppress the decomposition of Radiolabeled Compound (I) and retain it for a long period of time.

Boronic Acid Compound (II) can be produced by the method comprising the following Step 2 to Step 5. 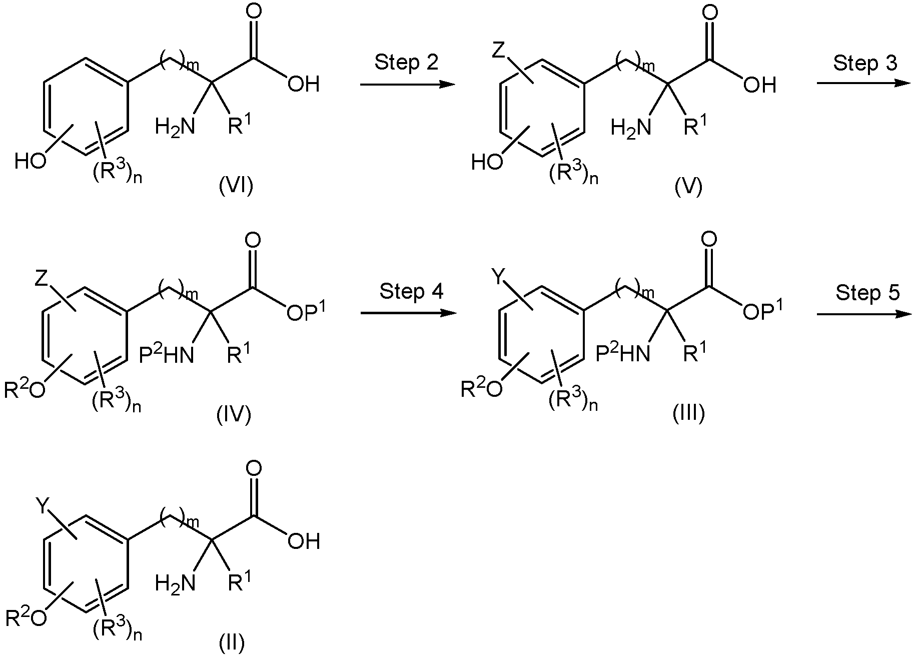 wherein Z is an iodine atom or a bromine atom, P¹ is a carboxy-protecting group, P² is an amino-protecting group, and the other symbols are as defined above.

In one embodiment, Z is preferably an iodine atom.

In one embodiment, P¹ is preferably a benzyl group or a C₁₋₂ alkyl group (a methyl group, an ethyl group), more preferably a benzyl group.

In one embodiment, P² is preferably a tert-butoxycarbonyloxy group or a benzyloxycarbonyl group, more preferably a tert-butoxycarbonyloxy group.

Step 2 is a step of subjecting a compound represented by Formula (VI) or a salt thereof (hereinafter also referred to as Compound (VI)) to iodination or bromination to obtain a compound represented by Formula (V) or a salt thereof (hereinafter also referred to as Compound (V)).

The iodination or bromination can be carried out by reacting Compound (VI) with an iodinating agent or brominating agent.

Examples of Compound (VI) include α-methyltyrosine, α-methyl-m-tyrosine, 4-hydroxy-α-methylphenylglycine, 3-hydroxy-α-methylphenylglycine, 2-amino-4-(4-hydroxyphenyl)-2-methylbutanoic acid, 2-amino-4-(3-hydroxyphenyl)-2-methylbutanoic acid and the like, which may be any of the L-, D-, or DL-form. Among them, α-methyltyrosine, or α-methyl-m-tyrosine is preferably used, and α-methyltyrosine is particularly preferably used. Compound (VI) may be a commercially available product or may be produced by a known method.

Examples of the iodinating agent include iodine, N-iodosuccinimide and the like.

Examples of the brominating agent include bromine, N-bromosuccinimide and the like.

The amount of the iodinating agent or brominating agent to be used is usually 1 to 5 mol, preferably 1 to 2 mol, per 1 mol of Compound (VI).

When iodine is used, the reaction is carried out in the presence of potassium iodide and concentrated ammonia (28%). The amount of the potassium iodide to be used is usually 0.5 to 5 mol, preferably 1 to 2 mol, per 1 mol of the iodine, and the amount of the concentrated ammonia to be used is usually 5 to 200 mol, preferably 20 to 50 mol, per 1 mol of the iodine.

The reaction is carried out usually in a solvent. The solvent to be used in the present invention is not particularly limited as long as it does not adversely affect the reaction. Examples thereof include water; alcohol solvents such as ethanol, methanol, isopropanol and the like; halogen solvents such as carbon tetrachloride and the like, and the like. These may be used in combination of two or more. Among them, water is preferably used.

The amount of the solvent to be used is usually 0.1 to 100-fold in volume relative to Compound (VI).

For example, when iodine is used, the reaction is preferably carried out by adding (preferably dropwise) a mixture of Compound (VI) and concentrated ammonia (and a solvent if necessary) to a mixture of iodine, potassium iodide and a solvent.

The reaction is carried out usually within the range of - 100 to 20°C, preferably -20 to 10°C. The reaction time varies depending on the reaction temperature, and it is usually about 30 min to about 24 hr, preferably about 1 to about 12 hr.

The completion of the reaction can be confirmed by thin-layer chromatography, liquid chromatography and the like.

After the completion of the reaction, Compound (V) can be isolated and/or purified from the reaction mixture by separation means such as concentration, crystallization, recrystallization, distillation, solvent extraction, fractional distillation, chromatography and the like, according to a conventional method.

By this reaction, an iodine atom or a bromine atom is introduced, for example, into the 3-position on the benzene ring in the case of α-methyltyrosine (the hydroxy group is at the 4-position on the benzene ring), or into the 4-position or 6-position on the benzene ring in the case of α-methyl-m-tyrosine (the hydroxy group is at the 3-position on the benzene ring).

Step 3 is a step of protecting the amino group and carboxy group of Compound (V) (introduction of P¹ and P²), and introducing R² to the hydroxy group to obtain a compound represented by Formula (IV) or a salt thereof (hereinafter also referred to as Compound (IV)).

The introduction of R² can carried out by reacting Compound (V) with the corresponding iodide or bromide in the presence of a base.

The amount of the corresponding iodide or bromide to be used is usually 1 to 5 mol, preferably 1 to 2 mol, per 1 mol of the substrate.

Examples of the base include potassium carbonate, sodium carbonate, sodium hydrogencarbonate, cesium carbonate and the like.

The amount of the base to be used is usually 1 to 5 mol, preferably 1 to 2 mol, per 1 mol of the substrate.

The reaction is carried out usually in a solvent. The solvent to be used in the present invention is not particularly limited as long as it does not adversely affect the reaction. Examples thereof include amide solvents such as dimethylformamide, dimethylacetamide, N-methylpyrrolidinone and the like; ether solvents such as tetrahydrofuran, 1,4-dioxane and the like, and the like. These may be used in combination of two or more. Among them, dimethylformamide is preferably used.

The amount of the solvent to be used is usually 0.1 to 100-fold in volume relative to the substrate.

The reaction is carried out usually within the range of 0 to 100°C, preferably room temperature to 50°C. The reaction time varies depending on the reaction temperature, and it is usually about 30 min to about 24 hr, preferably about 1 hr to about 17 hr.

The completion of the reaction can be confirmed by thin-layer chromatography, liquid chromatography and the like.

After the completion of the reaction, the target product can be isolated and/or purified from the reaction mixture by separation means such as concentration, crystallization, recrystallization, distillation, solvent extraction, fractional distillation, chromatography and the like, according to a conventional method.

The carboxy-protecting group (P¹) and amino-protecting group (P²) are selected so that the deprotection in Step 5 can be carried out under mild conditions where the introduced R² and the boryl group or its ester group are not eliminated.

In addition, it is particularly preferable to obtain Boronic Acid Compound (II) as a boronic acid pinacol ester (Y is a 4,4,5,5-tetramethyl-1,3,2-dioxaboran-2-yl group) because it can be obtained as a crystal, can be easily purified, and can be obtained with a higher purity. Therefore, it is desirable to select a protecting group that allows deprotection in Step 5 under conditions where Y is not hydrolyzed.

Preferred amino-protecting group is a tert-butoxycarbonyloxy group.

Preferred carboxy-protecting group is a benzyl group or a C₁₋₂ alkyl group (a methyl group, an ethyl group), and particularly preferred is a benzyl group.

The introduction of each protecting group can be carried out according to a method known per se.

Since the introduction of R² to the hydroxy group is carried out by reacting Compound (V) with the corresponding iodide or bromide, as described above, there is a risk that R² may also be introduced into the amino group at this time. Therefore, an amino protecting group (P²) is introduced before the introduction of R². Furthermore, when introducing R² as described above, there is a risk that R² may also be introduced into the carboxy group, so if desired, the desired carboxy protecting group (P¹) may be introduced after removing R².

Preferred combinations thereof include
a combination in which R² is a C₁₋₆ alkyl group (preferably a methyl group, an ethyl group, a propyl group) or a C₁₋₆ haloalkyl group (preferably a trifluoromethyl group, a 2-fluoroethyl group), the carboxy-protecting group (P¹) is a benzyl group, and the amino-protecting group (P²) is a tert-butoxycarbonyloxy group, and
a combination in which R² is a C₇₋₁₄ aralkyl group (preferably a benzyl group), the carboxy-protecting group (P¹) is a C₁₋₂ alkyl group (a methyl group, an ethyl group), and the amino-protecting group (P²) is a tert-butoxycarbonyloxy group.

After the completion of the reaction, Compound (IV) can be isolated and/or purified from the reaction mixture by separation means such as concentration, crystallization, recrystallization, distillation, solvent extraction, fractional distillation, chromatography and the like, according to a conventional method.

Among Compound (IV), a compound represented by the following Formula (IV') is a novel compound. 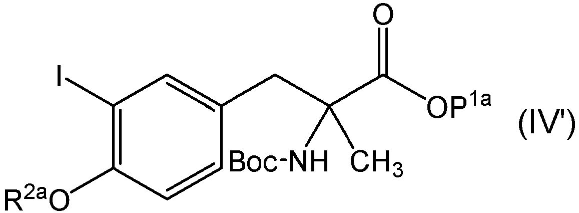 wherein R^{2a} is a C₁₋₃ alkyl group, and P^{1a} is a benzyl group or a C₁₋₃ alkyl group.

Step 4 is a step of reacting Compound (IV) with a reagent for introducing boronic acid, in the presence of a palladium catalyst and a base to obtain Boronic Acid Compound (III).

Examples of the reagent for introducing boronic acid include 4,4,4',4',5,5,5',5'-octamethyl-2,2'-bi-1,3,2-dioxaborolane (also known as bis(pinacolato)diboron), 4,4,5,5-trimethyl-1,3,2-dioxaborolane, tetrahydroxydiborane and the like. Among them, bis(pinacolato)diboron is preferably used. These reagents for introducing boronic acid may be commercially available products.

The amount of the reagent for introducing boronic acid to be used is usually 1 to 10 mol, preferably 1 to 3 mol, per 1 mol of Compound (IV).

Examples of the palladium catalyst include [1,1'-bis(diphenylphosphino)ferrocene]dichloropalladium(II) (PdCl₂(dppf)) or its dichloromethane adduct, palladium acetate, tetrakis(triphenylphosphine)palladium(0) (Pd(PPh₃)₄) and the like. Among them, PdCl₂(dppf) is preferably used.

The amount of the palladium catalyst to be used is usually 0.001 to 1 mol, preferably 0.01 to 0.2 mol, per 1 mol of Compound (IV).

Examples of the base include alkali metal acetates such as potassium acetate, sodium acetate and the like, and the like. Among them, an alkali metal acetate is preferably used, and potassium acetate is particularly preferably used.

The amount of the base to be used is usually 0.5 to 10 mol, preferably 1 to 5 mol, per 1 mol of Compound (IV).

The reaction is carried out usually in a solvent. The solvent to be used in the present invention is not particularly limited as long as it does not adversely affect the reaction. Examples thereof include sulfoxide solvents such as dimethyl sulfoxide and the like; amide solvents such as dimethylformamide, dimethylacetamide, N-methylpyrrolidinone and the like; and ether solvents such as tetrahydrofuran, 1,4-dioxane and the like. These may be used in combination of two or more. Among them, a sulfoxide solvent or an amide solvent is preferably used, and dimethyl sulfoxide is particularly preferably used.

The amount of the solvent to be used is usually 0.1 to 100-fold in volume relative to Compound (IV).

The reaction is preferably carried out, for example, by adding (preferably adding dropwise) a reagent for introducing boronic acid to a mixture of Compound (IV), palladium catalyst, base and solvent.

The reaction is carried out usually within the range of 0 to 200°C, preferably room temperature to 120°C. The reaction time varies depending on the reaction temperature, and it is usually about 10 min to about 48 hr, preferably about 1 to about 12 hr.

The completion of the reaction can be confirmed by thin-layer chromatography, liquid chromatography and the like.

After the completion of the reaction, Boronic Acid Compound (III) can be isolated and/or purified from the reaction mixture by separation means such as concentration, crystallization, recrystallization, distillation, solvent extraction, fractional distillation, chromatography and the like, according to a conventional method.

Boronic Acid Compound (III) is a novel compound.

Boronic Acid Compound (III) may be any of Boronic Acid Compound (IIIa) or Compound (IIIb) represented by the following formulae, and racemates thereof. 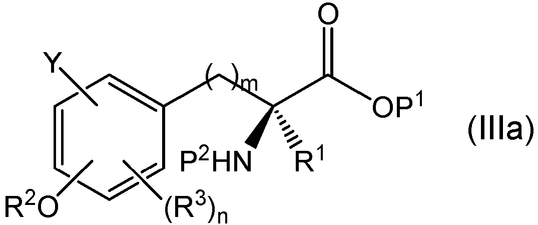 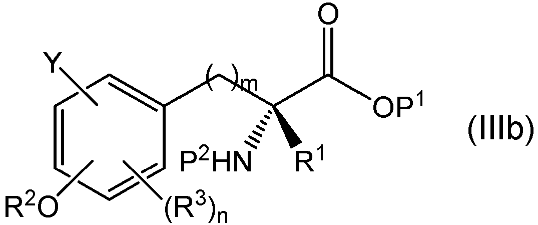 wherein each symbol is as defined above.

In one embodiment, Boronic Acid Compound (III) is preferably Boronic Acid Compound (IIIa).

Step 5 is a step of removing the carboxy-protecting group (P¹) and amino-protecting group (P²) of Boronic Acid Compound (III) to obtain Boronic Acid Compound (II).

The removal of each protecting group is carried out according to a method known per se. For example, when R² is a C₁₋₆ alkyl group (preferably a methyl group, an ethyl group, a propyl group) or a C₁₋₆ haloalkyl group (preferably a trifluoromethyl group, a 2-fluoroethyl group), and the carboxy-protecting group (P¹) is a benzyl group, then the removal of P¹ is carried out by subjecting Boronic Acid Compound (III) to catalytic hydrogenation.

In this case, when Y is a 4,4,5,5-tetramethyl-1,3,2-dioxaboran-2-yl group, then Y is not hydrolyzed, and therefore, Boronic Acid Compound (II) can be obtained as a boronic acid pinacol ester.

When R² is a C₇₋₁₄ aralkyl group (preferably a benzyl group), and the carboxy-protecting group (P¹) is a C₁₋₂ alkyl group (a methyl group, an ethyl group), then the removal of P¹ is carried out by treating Boronic Acid Compound (III) with a base such as lithium hydroxide, sodium hydroxide and the like.

When the amino-protecting group (P²) is a tert-butoxycarbonyl group, then the removal of P² is carried out by treating Boronic Acid Compound (III) with an acid such as trifluoroacetic acid, hydrogen chloride and the like.

Since all of the above removals of the protecting groups are carried out under mild conditions, elimination of R² and the (-B(OH)₂) or its ester group is unlikely to occur.

After the completion of the reaction, Boronic Acid Compound (II) can be isolated and/or purified from the reaction mixture by separation means such as concentration, crystallization, recrystallization, distillation, solvent extraction, fractional distillation, chromatography and the like, according to a conventional method.

The reaction conditions such as solvent and reaction temperature in each step in the production method of the present invention described above are described in detail as representative examples in Examples below, but are not necessarily limited thereto, and those skilled in the art can make appropriate selections based on their general knowledge in organic synthesis.

Thus-produced Radiolabeled Compound (I) bind specifically to LAT1, the expression of which is increased in tumor or cancer cells, and is then taken up into the cells and stably accumulated in the cells.

Since Radiolabeled Compound (I) targets tumor or cancer cells expressing LAT1, Radiolabeled Compound (I) comprising a therapeutically effective a radionuclide can be useful in the treatment of tumors or cancers expressing LAT1. Examples of such radionuclide include ²¹¹At, ²¹⁰At, ¹³¹I, ¹²⁵I and ⁷⁷Br.

Examples of the tumor or cancer expressing LAT1 include pancreatic cancer, leukemia, melanoma, colon cancer, lung cancer, prostate cancer, stomach cancer, breast cancer, kidney cancer, laryngeal cancer, esophageal cancer, liver cancer, lymphoma, myeloma, head and neck cancer, ovarian cancer, bladder cancer, childhood cancer, childhood leukemia, brain tumor (including primary brain tumor, metastatic brain tumor), osteosarcoma, soft tissue sarcoma, soft tissue tumor and the like.

In addition, Radiolabeled Compound (I) has higher retention at tumor or cancer sites, and has a level of clearance that does not cause side effects, and therefore can exhibit improved anti-tumor effects in vivo.

Furthermore, Radiolabeled Compound (I) can treat tumors or cancers expressing LAT1 with almost no side effects.

Furthermore, Radiolabeled Compound (I) is stable in blood and urine, and is therefore thought to be stable from its arrival at the tumor to its excretion.

Furthermore, since Radiolabeled Compound (I) can exhibit excellent anti-tumor effects even against tumors or cancers that have acquired resistance to anti-cancer drugs (for example, resistance to platinum preparations (e.g., Cisplatin), resistance to nucleic acid synthesis inhibitors (e.g., Gemcitabine)), the need for treatment strategies that are usually performed against tumors or cancers that has acquired resistance to anti-cancer drugs, such as increasing drug doses, changing drugs, or changing treatment methods, may be reduced.

The dose of Radiolabeled Compound (I) used for therapeutic purposes is usually determined depending on the type of radionuclide used, the patient's weight, age and sex, the therapeutic site, and the like. For example, in the case of humans, the estimated effective dose of Radiolabeled Compound (I) with ²¹¹At is approximately 100 MBq to 900 MBq.

Radiolabeled Compound (I) are usually mixed with a pharmaceutically acceptable carrier and used as a pharmaceutical composition. Pharmaceutically acceptable carriers refer to a biocompatible solution with due consideration for sterility, pH, isotonicity, stability, etc., and may include any and all solvents, diluents (including sterile saline, sodium chloride injection, Ringer's solution, dextrose injection, dextrose and sodium chloride injection, lactated Ringer's solution, and other aqueous buffers), dispersants, coatings, antibacterial and antifungal agents, isotonic agents, and the like. Pharmacologically acceptable carriers can also contain stabilizers, preservatives, antioxidants, or other additives known to those skilled in the art.

The dosage form of the pharmaceutical composition is not particularly limited, and it can be prepared as a pharmaceutical composition for oral administration in the form of granules, fine granules, powders, hard capsules, soft capsules, syrups, emulsions, suspensions, liquids and the like; or for parenteral administration such as intravenous administration, intramuscular administration and subcutaneous administration, in the form of injections, drip infusions, transdermal absorptions, transmucosal absorptions, nasal drops, inhalations, suppositories, and the like. These formulations can be prepared according to conventional methods. Preferred are liquid formulations for oral administration or for injection.

Such liquid formulations are prepared by dissolving Radiolabeled Compound (I) in water, or may also be prepared by dissolving Radiolabeled Compound (I) in saline or glucose solution. Buffers or preservatives may be added as necessary. As described above, a reducing agent such as ascorbic acid, an alkali metal ascorbate and an alkaline-earth metal ascorbate can also be included. In particular, for the production of injections, the active ingredient is dissolved in distilled water for injection, together with a pH adjuster such as hydrochloric acid, sodium hydroxide, lactose, sodium lactate, sodium monohydrogen phosphate and sodium dihydrogen phosphate, and an isotonic agent such as sodium chloride and glucose, as needed, and then the mixture is sterilely filtered and filled into an ampule to prepare an injection. Mannitol, dextrin, cyclodextrin, gelatin and the like may also be further added, and the mixture is vacuum lyophilized to prepare an injection that is dissolved immediately before use. Furthermore, lecithin, polysorbate 80, polyoxyethylene hydrogenated castor oil and the like may be added to the active ingredient, and the mixture is emulsified in water to prepare an emulsion for injection.

The half-life of the radionuclide contained in Radiolabeled Compound (I) is short; it is 7.2 hours for ²¹¹At, 8.3 hours for ²¹⁰At, 8.04 days for ¹³¹I, 59.4 days for ¹²⁵I, and 57 hours for ⁷⁷Br. Therefore, it is desirable to prepare a pharmaceutical composition immediately before administration to the subject so that it contains the amount of Radiolabeled Compound (I) required for administration.

Since Boronic Acid Compound (II) is also a boron-containing compound that targets tumor or cancer cells expressing LAT1, it may be useful as a drug for boron neutron capture therapy (BNCT) in combination with neutron irradiation. This therapy utilizes the property that boron (¹⁰B), which naturally exists at approximately 20%, has a large capture cross section for thermal neutrons, and destroys cancer cells with helium nuclei (⁴He²⁺) and lithium nuclei (⁷Li⁺) released by the nuclear reaction between ¹⁰B and thermal neutrons. Both the helium nuclei and the lithium nuclei have high linear energy and a short range close to the cell size. Therefore, by irradiating Boronic Acid Compound (II) taken up into tumor or cancer cells with neutrons, only the tumor or cancer cells can be effectively destroyed.

### [Examples]

The present invention will be explained in detail by the following Examples, which are merely examples and are not intended to limit the present invention and can be modified without departing from the scope of the present invention.

In the following Examples, the radiochemical yield was calculated using the following formula. Radiochemical yield (%) = (radioactivity of the desired compound on thin-layer plate/total radioactivity on thin- layer plate) × 100

### Example 1

### Synthesis of (S)-3-(4,4,5,5-tetramethyl-1,3,2-dioxaboran-2-yl)-O-methyl-α-methyltyrosine hydrochloride (6) (Compound (6) Bpin-AMT-OMe)

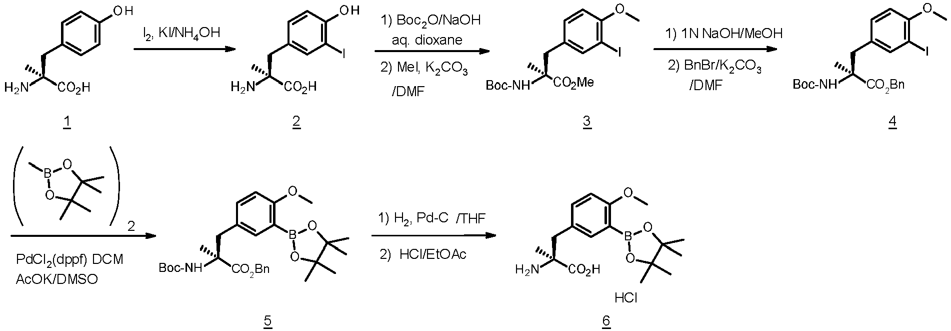

### a) Synthesis of (S)-3-iodo-α-methyltyrosine (2) (Compound (2))

To a mixture of potassium iodide (5.3 g, 32.1 mmol) and water (14 mL) was added iodine (7.1 g, 28.1 mmol), and the mixture was stirred for 2 hr, and added to a mixture (cooled to -7°C) of (S)-α-methyltyrosine (1) (5.2 g, 26.8 mmol), concentrated aqueous ammonia (28%, 62 mL) and water (8 mL) at - 5°C or lower, and the mixture was stirred at -7 to -5°C for 3 hr. To the reaction solution was added 15% aqueous sodium sulfite solution (15 mL), and the mixture was allowed to warm to room temperature, adjusted to pH 6.5 to 7 with 6M hydrochloric acid under ice-cooling, and stirred under ice-cooling for 1 hr. The precipitated solid was collected by filtration, washed with cold water and acetone, and dried under reduced pressure to give Compound (2) as a white solid (7.4 g, yield 86%).
¹H-NMR (300MHz, DMSO-d₆+TFA, TMS): 8.25(3H, br), 7.51 (1H, d, J=2.1 Hz), 7.03 (1H, dd, J=2.1, 8.1 Hz), 6.84 (1H, d, J=8.1 Hz), 3.03 (1H, d, J=14.1 Hz), 2.87 (1H, d, J=14.1 Hz), 1.44

### (3H, s)

### b) Synthesis of (S)-N-Boc-3-iodo-O-methyl-α-methyltyrosine methyl ester (3) (Compound (3))

To a mixture of Compound (2) (4.3 g, 13.4 mmol), 1M aqueous sodium hydroxide solution (13 mL) and 1,4-dioxane (20 mL) was added di-t-butyl dicarbonate (8.8 g, 40.2 mmol), and the mixture was stirred at 60°C for 20 hr. The reaction solution was allowed to cool to room temperature, and concentrated under reduced pressure. To the residue were added water (30 mL) and methyl t-butyl ether-heptane (1:2, 60 mL), and the mixture was subjected to extraction by liquid separation. The organic layer was subjected to back-extraction with 5% aqueous sodium hydrogencarbonate solution (20 mL) and water (20 mL). The combined aqueous layer was adjusted to pH 1-2 with 0.5M aqueous sodium hydrogen sulfate solution, and subjected to extraction with methyl t-butyl ether-ethyl acetate (1:1, 60 mL). The extract was washed with water and saturated brine. The solvent was evaporated under reduced pressure to give a pale-brown solid (2.9 g). The obtained pale-brown solid (2.9 g) was dissolved in N,N-dimethylformamide (20 mL), potassium carbonate (2.1 g, 15 mmol) and methyl iodide (2.1 g, 15 mmol) were added, and the mixture was stirred at room temperature for 17 hr. Ethyl acetate (60 mL) was added, the mixture was washed with water (60 mL) and saturated brine (30 mL), and the solvent was evaporated under reduced pressure. The residue was purified by silica gel column chromatography to give Compound (3) as a white amorphous (2.6 g, yield 43%). ¹H-NMR (300MHz, CDCl₃, TMS): 7.50 (1H, d, J=2.1 Hz), 7.00 (1H, dd, J=2.1, 8.4 Hz), 6.71 (1H, d, J=8.4 Hz), 5.17 (1H, brs), 3.85 (3H, s), 3.77 (3H, s), 3.33 (1H, d, J=13.5 Hz), 3.10 (1H, d, J=13.5 Hz), 1.55 (3H, s), 1.49 (9H, s)

### c) Synthesis of (S)-N-Boc-3-iodo-O-methyl-α-methyltyrosine benzyl ester (4) (Compound (4))

To a mixture of Compound (3) (2.6 g, 5.8 mmol) in methanol (7 mL) and tetrahydrofuran (13 mL) was added 1M aqueous sodium hydroxide solution (7.0 mL, 7.0 mmol), and the mixture was stirred at room temperature for 4 hr, and then at 60°C for 19 hr. The reaction solution was allowed to cool to room temperature, and concentrated. To the residue were added water (30 mL) and methyl t-butyl ether (30 mL), and the mixture was subjected to liquid separation. The aqueous layer was adjusted to pH 2-3 with 0.5M aqueous sodium hydrogen sulfate solution, and subjected to extraction with methyl t-butyl ether (30 mL). The extract was washed with water, and the solvent was evaporated under reduced pressure to give a white amorphous (2.3 g). The obtained white amorphous (2.3 g, 5.3 mmol) was dissolved in N,N-dimethylformamide (14 mL), potassium carbonate (880 mg, 6.3 mmol) and benzyl bromide (0.75 mL, 6.3 mmol) were added, and the mixture was stirred at 50°C for 4 hr, and allowed to cool to room temperature. Ethyl acetate (30 mL) was added, the mixture was washed with water and saturated brine, and the solvent was evaporated under reduced pressure. The residue was purified by silica gel column chromatography to give Compound (4) as a white amorphous (2.4 g, yield 79%). ¹H-NMR (300MHz, DMSO-d₆, TMS): 7.52-7.30 (7H, m), 7.00 (1H, dd, J=2.1, 8.4 Hz), 6.92 (1H, d, J=8.4 Hz), 5.12 (1H, d, J=12.6 Hz), 5.03 (1H, d, J=12.6 Hz), 3.79 (3H, s), 3.28 (1H, d, J=15.0 Hz), 2.72 (1H, d, J=15.0 Hz), 1.42 (9H, s), 1.16 (3H, s)

### d) Synthesis of (S)-N-Boc-3-(4,4,5,5-tetramethyl-1,3,2-dioxaboran-2-yl)-O-methyl-α-methyltyrosine benzyl ester (5) (Compound (5))

Under nitrogen atmosphere, to a mixture of Compound (4) (2.4 g, 4.6 mmol), [1,1'-bis(diphenylphosphino)ferrocene]dichloropalladium(II) dichloromethane adduct (0.29 g, 0.35 mmol), potassium acetate (1.79 g, 18.3 mmol) and dimethyl sulfoxide (19 mL) was added bis(pinacolato)diboron (2.32 g, 9.1 mmol), and the mixture was stirred at room temperature for 1 hr, and then stirred at 55°C for 2 hr. The reaction solution was allowed to cool to room temperature, methyl t-butyl ether (50 mL) and water (50 mL) were added, and the mixture was filtered through Celite, and subjected to liquid separation. The aqueous layer was subjected to extraction with methyl t-butyl ether (30 mL), the organic layers were combined, and washed with water (30 mL), saturated brine (30 mL), and the solvent was evaporated under reduced pressure. The residue was purified by silica gel column chromatography to give Compound (5) as a white amorphous (1.6 g, yield 67%).
¹H-NMR (300MHz, CDCl₃, TMS): 7.5-7.3 (6H, m), 7.06 (1H, dd, J=2.1, 8.4 Hz), 6.72 (1H, d, J=8.4 Hz), 5.21 (1H, d, J=12.6 Hz), 5.13 (1H, d, J=12.6 Hz), 3.80 (3H, s), 3.31 (1H, d, J=13.5 Hz), 3.16 (1H, d, J=13.5 Hz), 1.46 (9H, s), 1.32 (12H, s), 1.26

### (3H, s)

### e) Synthesis of (S)-3-(4,4,5,5-tetramethyl-1,3,2-dioxaboran-2-yl)-O-methyl-α-methyltyrosine hydrochloride (6) (Compound (6) Bpin-AMT-OMe)

A mixture of Compound (5) (1.3 g, 2.5 mmol), 10% palladium-carbon (55% hydrous, 0.33 g) and tetrahydrofuran (13 mL) was stirred under hydrogen gas atmosphere for 3 hr. The reaction solution was filtered through Celite, and the solvent was evaporated under reduced pressure to give a white amorphous (1.1 g). To a mixture of the obtained amorphous (1.1 g, 2.5 mmol) and ethyl acetate (4.0 mL) was added 4M hydrochloric acid-ethyl acetate solution (4 mL), and the mixture was stirred at room temperature for 18 hr. The precipitated solid was collected by filtration, and washed with ethyl acetate to give Compound (6) as a white powder (0.51 g, yield 55%).
¹H-NMR (300MHz, DMSO-d₆, TMS): 8.35 (3H, br), 7.39 (1H, d, J=2.4 Hz), 7.28 (1H, dd, J=2.4, 8.4 Hz), 6.94 (1H, d, J=8.4 Hz), 3.72 (3H, s), 3.08 (1H, d, J=13.8 Hz), 2.98 (1H, d, J=13.8 Hz), 1.46 (3H, s), 1.27 (12H, s)

### HPLC analysis condition

sample solution: 1 mg of Compound (6) was dissolved in 67% acetonitrile-containing water (1 mL) to prepare a sample solution.
detector: UV 275 nm
column: YMC-Pack Pro C18 RS (4.6 mmφ×25 cm, 5 µm, YMC)
column temperature: 30°C
mobile phase: H₂O (0.1% TFA)/MeCN (0.1% TFA)
mode: gradient
   0 min H₂O (0.1% TFA)/MeCN (0.1% TFA) = 95/5
   30 min H₂O (0.1% TFA)/MeCN (0.1% TFA) = 5/95
   40 min H₂O (0.1% TFA)/MeCN (0.1% TFA) = 5/95
flow rate: 1 mL/min
injection volume: 5 µL
analysis time: 40 min
retention time: Compound (6) 8.1 min

The HPLC chart is shown in Fig.1.

### Reference Example 1

### Preparation of ²¹¹At aqueous solution

According to the method described in Reference Example 1 of Patent Literature 2, an ²¹¹At aqueous solution was prepared. That is, ²¹¹At was produced by ²⁰⁹Bi(α,2n)²¹¹At nuclear reaction resulting from the irradiation of bismuth with helium particles accelerated (28 MeV) by a cyclotron. After the irradiation, by heating, the target substance ²⁰⁹Bi was melted, while the ²¹¹At was vaporized. The vaporized ²¹¹At was collected to a cooled trap, and dissolved in a small amount of water to prepare an ²¹¹At aqueous solution.

### Example 2

### Synthesis of (S)-3-astato(²¹¹At)-O-methyl-α-methyltyrosine (²¹¹At-AAMT-OMe)

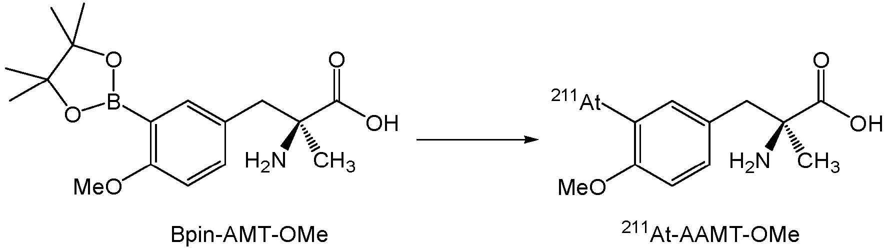

Compound (6) (Bpin-AMT-OMe) was dissolved in Meylon (7%) to prepare an aqueous solution (1 mg/mL). This aqueous solution (0.1 mL) was put in a 1.5 mL polypropylene (PP) tube. Then, the ²¹¹At aqueous solution (100 µL, 10 MBq, the radiation dose was measured using a curie meter (IGC-7, Hitachi)) prepared in Reference Example 1 and 0.1M KI aqueous solution (20 µL) were added, followed by sterile distilled water to bring the total volume to 200 µL. The mixture was reacted at 50°C for 45 min to give the crude product solution of (S)-3-astato (²¹¹At) -O-methyl-α-methyltyrosine (²¹¹At-AAMT-OMe).

The crude product solution (1 µL) was analyzed by thin-layer chromatography (TLC). The sample was spotted on a thin-layer plate (silica gel 60F₂₅₄), and a mixed solvent of acetonitrile/water (2/1) was used as a developing solvent. The thin-layer plate was exposed to an imaging plate (BAS IP MS 2025E, GE Healthcare) for about 15 min, and then analyzed by a bioimage analyzer (Tyhoon^{™} FLA-7000, Cytiva). The results are shown in Fig.2. ²¹¹At-AAMT-OMe in the crude product solution was detected at Rf 0.80, and the radiochemical yield (RCY) was 81.3%.

The crude product solution was injected into an HLB column (Oasis, WAT-186005125) equilibrated with sterile distilled water (1 mL) to capture the target product in the cartridge, and sterile distilled water was passed through the cartridge to elute impurities. Next, 30% ethanol solution (500 µL) was passed through the cartridge to elute the target product into sodium ascorbate aqueous solution (500 µL, final concentration 1%), thus obtaining the purified product. The radiochemical yield and radiochemical purity of the purified product were 81% and 98%, respectively.

Before purification, immediately after production, 3 hours after production, and 24 hours after production, ²¹¹At-AAMT-OMe was analyzed by thin-layer chromatography (TLC) in the same manner as described above. The results are shown in Fig.3. As shown in Fig.3, no decomposition of ²¹¹At-AAMT-OMe was observed even 24 hours after purification.

### Example 3

### Synthesis of (R)-3-(4,4,5,5-tetramethyl-1,3,2-dioxaboran-2-yl)-O-methyl-α-methyltyrosine hydrochloride (12) (Compound (12) (R)-Bpin-AMT-OMe)

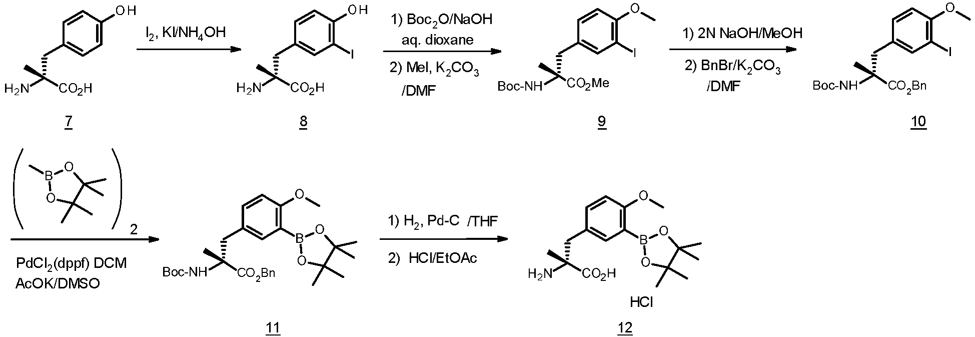

### a) Synthesis of (R)-3-iodo-α-methyltyrosine (8) (Compound (8))

To a mixture of potassium iodide (5.1 g, 30.7 mmol) and water (14 mL) was added iodine (6.8 g, 26.9 mmol), and the mixture was stirred for 2 hr, and added to a mixture (cooled to -7°C) of (R)-α-methyltyrosine (7) (5.0 g, 25.6 mmol), concentrated aqueous ammonia (28%, 59 mL) and water (8 mL) at - 5°C or lower, and the mixture was stirred at -7 to -5°C for 3 hr. To the reaction was added 15% aqueous sodium sulfite solution (15 mL), and the mixture was allowed to warm to room temperature, adjusted to pH 6.5 to 7 with 6M hydrochloric acid under ice-cooling, and stirred under ice-cooling for 1 hr. The precipitated solid was collected by filtration, washed with cold water and acetone, and dried under reduced pressure to give Compound (8) as a white solid (7.1 g, yield 86%).
¹H-NMR (400MHz, DMSO-d₆+TFA, TMS): 8.25(3H, br), 7.51 (1H, d, J=2.0 Hz), 7.03 (1H, dd, J=2.0, 8.4 Hz), 6.84 (1H, d, J=8.4 Hz), 3.03 (1H, d, J=14.1 Hz), 2.87 (1H, d, J=14.1 Hz), 1.44 (3H, s)

### b) Synthesis of (R)-N-Boc-3-iodo-O-methyl-α-methyltyrosine methyl ester (9) (Compound (9))

To a mixture of Compound (8) (4.3 g, 13.4 mmol), 1M aqueous sodium hydroxide solution (13 mL) and 1,4-dioxane (20 mL) was added di-t-butyl dicarbonate (8.8 g, 40.2 mmol), and the mixture was stirred at 60°C for 20 hr. The reaction solution was allowed to cool to room temperature, and concentrated under reduced pressure. To the residue were added water (30 mL) and methyl t-butyl ether-heptane (1:2, 60 mL), and the mixture was subjected to extraction by liquid separation. The organic layer was washed with 5% aqueous sodium hydrogencarbonate solution (20 mL) and water (20 mL). The combined washing aqueous layer was adjusted to pH 1-2 with 0.5M aqueous sodium hydrogen sulfate solution, and subjected to extraction with methyl t-butyl ether-ethyl acetate (1:1, 60 mL). The extract was washed with water and saturated brine. The solvent was evaporated under reduced pressure to give a pale-brown solid (4.2 g). The obtained pale-brown solid (4.2 g) was dissolved in N,N-dimethylformamide (20 mL), potassium carbonate (3.0 g, 21.9 mmol) and methyl iodide (3.1 g, 21.9 mmol) were added, and the mixture was stirred at room temperature for 17 hr. Ethyl acetate (60 mL) was added, the mixture was washed with water (60 mL) and saturated brine (30 mL), and the solvent was evaporated under reduced pressure. The residue was purified by silica gel column chromatography to give Compound (9) as a white amorphous (3.2 g, yield 53%). ¹H-NMR (400MHz, CDCl₃, TMS): 7.50 (1H, d, J=2.0 Hz), 7.00 (1H, dd, J=2.0, 8.4 Hz), 6.71 (1H, d, J=8.4 Hz), 5.17 (1H, brs), 3.85 (3H, s), 3.77 (3H, s), 3.33 (1H, d, J=13.6 Hz), 3.10 (1H, d, J=13.6 Hz), 1.55 (3H, s), 1.49 (9H, s)

### c) Synthesis of (R)-N-Boc-3-iodo-O-methyl-α-methyltyrosine benzyl ester (10) (Compound (10))

To a mixture of Compound (9) (3.2 g, 7.1 mmol) in methanol (13 mL) and tetrahydrofuran (15 mL) was added 2M aqueous sodium hydroxide solution (12.8 mL, 25.6 mmol), and the mixture was stirred at 60°C for 24 hr. The reaction solution was allowed to cool to room temperature, and concentrated. To the residue were added water (30 mL) and methyl t-butyl ether (30 mL), and the mixture was subjected to liquid separation. The aqueous layer was adjusted to pH 2-3 with 0.5M aqueous sodium hydrogen sulfate solution, and subjected to extraction with methyl t-butyl ether (30 mL). The extract was washed with water, and the solvent was evaporated under reduced pressure to give a white amorphous (3.0 g). The obtained white amorphous (3.0 g, 6.9 mmol) was dissolved in N,N-dimethylformamide (20 mL), potassium carbonate (1.2 g, 8.5 mmol) and benzyl bromide (1.0 mL, 8.5 mmol) were added, and the mixture was stirred at 50°C for 4 hr, and allowed to cool to room temperature. Ethyl acetate (30 mL) was added, the mixture was washed with water (30 mL) and saturated brine (30 mL), and the solvent was evaporated under reduced pressure. The residue was purified by silica gel column chromatography to give Compound (10) as a white amorphous (3.5 g, yield 94%).
¹H-NMR (400MHz, DMSO-d₆, TMS): 7.52-7.30 (7H, m), 7.00 (1H, dd, J=2.0, 8.4 Hz), 6.92 (1H, d, J=8.4 Hz), 5.12 (1H, d, J=12.4 Hz), 5.03 (1H, d, J=12.4 Hz), 3.79 (3H, s), 3.28 (1H, d, J=13.6 Hz), 2.72 (1H, d, J=13.6 Hz), 1.42 (9H, s), 1.16 (3H, s)

### d) Synthesis of (R)-N-Boc-3-(4,4,5,5-tetramethyl-1,3,2-dioxaboran-2-yl)-O-methyl-α-methyltyrosine benzyl ester (11) (Compound (11))

Under nitrogen atmosphere, to a mixture of Compound (10) (3.5 g, 6.6 mmol), [1,1'-bis(diphenylphosphino)ferrocene]dichloropalladium(II) dichloromethane adduct (0.41 g, 0.51 mmol), potassium acetate (2.62 g, 26.7 mmol) and dimethyl sulfoxide (26 mL) was added bis(pinacolato)diboron (3.38 g, 13.3 mmol), and the mixture was stirred at room temperature for 1 hr, and then stirred at 55°C for 2 hr. The reaction solution was allowed to cool to room temperature, methyl t-butyl ether (50 mL) and water (50 mL) were added, and the mixture was filtered through Celite, and subjected to liquid separation. The aqueous layer was subjected to extraction with methyl t-butyl ether (30 mL), and the organic layers were combined, and washed with water (30 mL), saturated brine (30 mL), and the solvent was evaporated under reduced pressure. The residue was purified by silica gel column chromatography to give Compound (11) as a white amorphous (1.7 g, yield 72 %). ¹H-NMR (400MHz, CDCl₃, TMS): 7.5-7.3 (6H, m), 7.06 (1H, dd, J=2.0, 8.4 Hz), 6.72 (1H, d, J=8.4 Hz), 5.21 (1H, d, J=12.4 Hz), 5.13 (1H, d, J=12.4 Hz), 3.80 (3H, s), 3.31 (1H, d, J=13.6 Hz), 3.16 (1H, d, J=13.6 Hz), 1.46 (9H, s), 1.32 (12H, s), 1.26 (3H, s)

### e) Synthesis of (R)-3-(4,4,5,5-tetramethyl-1,3,2-dioxaboran-2-yl)-O-methyl-α-methyltyrosine hydrochloride (12) (Compound (12) (R)-Bpin-AMT-OMe)

A mixture of Compound (11) (1.7 g, 3.2 mmol), 10% palladium-carbon (55% hydrous, 0.42 g) and tetrahydrofuran (17 mL) was stirred under hydrogen gas atmosphere for 3 hr. The reaction solution was filtered through Celite, and the solvent was evaporated under reduced pressure to give a white amorphous (1.4 g). To a mixture of the obtained amorphous (1.4 g, 3.2 mmol) and ethyl acetate (5.0 mL) was added 4M hydrochloric acid-ethyl acetate solution (5 mL), and the mixture was stirred at room temperature for 17 hr. The precipitated solid was collected by filtration, and washed with ethyl acetate to give a white powder (0.66 g). The powder was washed by suspension with ethyl acetate (15.0 mL) at room temperature for 1 hr. The solid was collected by filtration, and washed with ethyl acetate to give Compound (12) as a white powder (0.53 g, yield 44%).
¹H-NMR (400MHz, DMSO-d₆, TMS): 8.35 (3H, br), 7.39 (1H, d, J=2.4 Hz), 7.28 (1H, dd, J=2.4, 8.4 Hz), 6.94 (1H, d, J=8.4 Hz), 3.72 (3H, s), 3.08 (1H, d, J=14.0 Hz), 2.98 (1H, d, J=14.0 Hz), 1.46 (3H, s), 1.27 (12H, s)

### HPLC analysis condition

sample solution: 1 mg of Compound (12) was dissolved in 67% acetonitrile-containing water (1 mL) to prepare a sample solution.
detector: UV 275 nm
column: YMC-Pack Pro C18 RS (4.6 mmφ×25 cm, 5 µm, YMC)
column temperature: 30°C
mobile phase: H₂O (0.1% TFA)/MeCN (0.1% TFA)
mode: gradient
   0 min H₂O (0.1% TFA)/MeCN (0.1% TFA) = 95/5
   30 min H₂O (0.1% TFA)/MeCN (0.1% TFA) = 5/95
   40 min H₂O (0.1% TFA)/MeCN (0.1% TFA) = 5/95
flow rate: 1 mL/min
injection volume: 5 µL
analysis time: 40 min
retention time: Compound (12) 7.3 min

The HPLC chart is shown in Fig.4.

### Example 4

### Synthesis of (R)-3-astato(²¹¹At)-O-methyl-α-methyltyrosine ((R)-²¹¹At-AAMT-OMe)

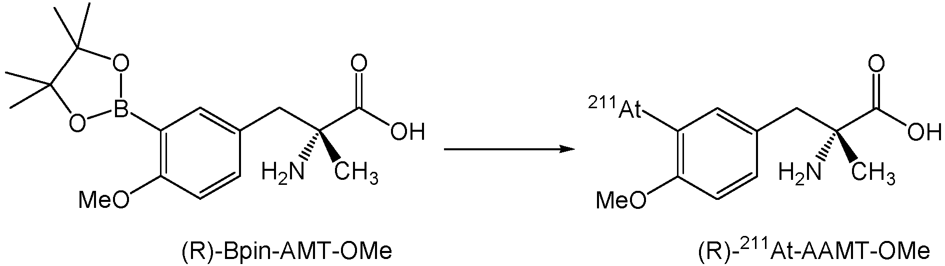

Compound (12) ((R)-Bpin-AMT-OMe) was dissolved in Meylon (7%) to prepare an aqueous solution (1 mg/mL). This aqueous solution (0.1 mL) was put in a 1.5 mL polypropylene (PP) tube. Then, the ²¹¹At aqueous solution (0.1 mL, 5 MBq, the radiation dose was measured using a curie meter (IGC-7, Hitachi)) prepared in Reference Example 1 and 0.1M KI aqueous solution (20 µL) were added, followed by sterile distilled water to bring the total volume to 200 µL. The mixture was reacted at 50°C for 45 min to give the crude product solution of (R)-3-astato (²¹¹At) -O-methyl-α-methyltyrosine ((R)-²¹¹At-AAMT-OMe).

The crude product solution (1 µL) was analyzed by thin-layer chromatography (TLC). The sample was spotted on a thin-layer plate (silica gel 60F₂₅₄), and a mixed solvent of acetonitrile/water (2/1) was used as a developing solvent. The thin-layer plate was exposed to an imaging plate (BAS IP MS 2025E, GE Healthcare) for about 15 min, and then analyzed by a bioimage analyzer (Tyhoon^{™} FLA-7000, Cytiva) . (R)-²¹¹At-AAMT-OMe in the crude product solution was detected at Rf 0.80, and the radiochemical yield (RCY) was 79.6%.

The crude product solution was injected into an HLB column (Oasis, WAT-186005125) equilibrated with sterile distilled water (1 mL) to capture the target product in the cartridge, and sterile distilled water was passed through the cartridge to elute impurities. Next, 30% ethanol solution (500 µL) was passed through the cartridge to elute the target product into sodium ascorbate aqueous solution (500 µL, final concentration 1%), thus obtaining the purified product. This purified product was analyzed by thin-layer chromatography (TLC) in the same manner as described above. The results are shown in Fig.5. The radiochemical yield and radiochemical purity of the purified product were 79.6% and 99.9%, respectively.

24 hours after production, (R)-²¹¹At-AAMT-OMe was analyzed by thin-layer chromatography (TLC) in the same manner as described above. The results are shown in Fig.6. As shown in Fig.6, no decomposition of (R)-²¹¹At-AAMT-OMe was observed even 24 hours after purification.

### Example 5

### Synthesis of (S)-3-(4,4,5,5-tetramethyl-1,3,2-dioxaboran-2-yl)-O-ethyl-α-methyltyrosine hydrochloride (18) (Compound (18) Bpin-AMT-OEt)

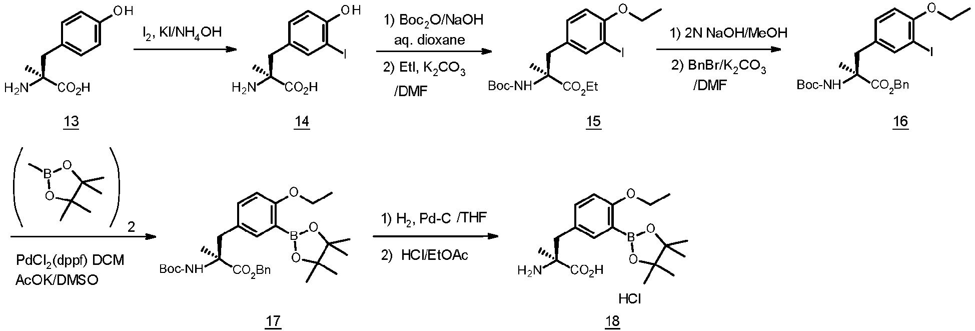

### a) Synthesis of (S)-3-iodo-α-methyltyrosine (14) (Compound (14))

To a mixture of potassium iodide (5.3 g, 32.1 mmol) and water (14 mL) was added iodine (7.1 g, 28.1 mmol), and the mixture was stirred for 2 hr, and added to a mixture (cooled to -7°C) of (S)-α-methyltyrosine (13) (5.0 g, 25.6 mmol), concentrated aqueous ammonia (28%, 62 mL) and water (8 mL) at - 5°C or lower, and the mixture was stirred at -7 to -5°C for 3 hr. To the reaction solution was added 15% aqueous sodium sulfite solution (15 mL), and the mixture was allowed to warm to room temperature, adjusted to pH 6.5 to 7 with 6M hydrochloric acid under ice-cooling, and stirred under ice-cooling for 1 hr. The precipitated solid was collected by filtration, washed with cold water and acetone, and dried under reduced pressure to give Compound (14) as a white solid (8.4 g, yield 98%).
¹H-NMR (400MHz, DMSO-d₆+TFA, TMS): 8.25(3H, br), 7.51 (1H, d, J=2.0 Hz), 7.03 (1H, dd, J=2.0, 8.4 Hz), 6.84 (1H, d, J=8.4 Hz), 3.03 (1H, d, J=14.1 Hz), 2.87 (1H, d, J=14.1 Hz), 1.44 (3H, s)

### b) Synthesis of (S)-N-Boc-3-iodo-O-ethyl-α-methyltyrosine ethyl ester (15) (Compound (15))

To a mixture of Compound (14) (4.3 g, 13.4 mmol), 1M aqueous sodium hydroxide solution (31 mL), 1,4-dioxane (50 mL) and water (25 mL) was added di-t-butyl dicarbonate (3.3 g, 14.7 mmol), and the mixture was stirred at 60°C for 22 hr. The reaction solution was allowed to cool to room temperature, and concentrated under reduced pressure. To the residue were added water (30 mL) and methyl t-butyl ether-heptane (1:2, 60 mL), and the mixture was subjected to extraction by liquid separation. The organic layer was washed with 5% aqueous sodium hydrogencarbonate solution (20 mL) and water (20 mL). The combined washing aqueous layer was adjusted to pH 1-2 with 0.5M aqueous sodium hydrogen sulfate solution, and subjected to extraction with methyl t-butyl ether-ethyl acetate (1:1, 60 mL). The extract was washed with water and saturated brine. The solvent was evaporated under reduced pressure to give a pale-brown solid (3.1 g). The obtained pale-brown solid (3.1 g) was dissolved in N,N-dimethylformamide (20 mL), potassium carbonate (2.1 g, 15 mmol) and ethyl iodide (2.4 g, 15 mmol) were added, and the mixture was stirred at room temperature for 16 hr. Ethyl acetate (60 mL) was added, the mixture was washed with water (60 mL) and saturated brine (30 mL), and the solvent was evaporated under reduced pressure. The residue was purified by silica gel column chromatography to give Compound (15) as a white amorphous (3.0 g, yield 47%).
¹H-NMR (400MHz, CDCl₃, TMS): 7.50 (1H, d, J=2.0 Hz), 6.99 (1H, dd, J=2.0, 8.4 Hz), 6.68 (1H, d, J=8.4 Hz), 5.18 (1H, brs), 4.21 (2H, q, J=6.8 Hz), 4.06 (2H, q, J=6.8 Hz), 3.33 (1H, d, J=13.2 Hz), 3.10 (1H, d, J=13.2 Hz), 1.54-1.57 (3H, m), 1.49 (9H, s), 1.46 (3H, t, J=6.8 Hz), 1.32 (3H, t, J=6.8 Hz)

### c) Synthesis of (S)-N-Boc-3-iodo-O-ethyl-α-methyltyrosine benzyl ester (16) (Compound (16))

To a mixture of Compound (15) (3.0 g, 6.3 mmol) in methanol (15 mL) and tetrahydrofuran (15 mL) was added 2M aqueous sodium hydroxide solution (11.3 mL, 22.6 mmol), and the mixture was stirred at 60°C for 17 hr. The reaction solution was allowed to cool to room temperature, and concentrated. To the residue were added water (30 mL) and methyl t-butyl ether (30 mL), and the mixture was subjected to liquid separation. The aqueous layer was adjusted to pH 2-3 with 0.5M aqueous sodium hydrogen sulfate solution, and subjected to extraction with methyl t-butyl ether (30 mL). The extract was washed with water, and the solvent was evaporated under reduced pressure to give a white amorphous (2.9 g). The obtained white amorphous (2.9 g, 6.3 mmol) was dissolved in N,N-dimethylformamide (17 mL), potassium carbonate (1.0 g, 7.6 mmol) and benzyl bromide (0.87 mL, 7.6 mmol) were added, and the mixture was stirred at 50°C for 3 hr, and allowed to cool to room temperature. Ethyl acetate (30 mL) was added, and the mixture was washed with water and saturated brine, and the solvent was evaporated under reduced pressure. The residue was purified by silica gel column chromatography to give Compound (16) as a white amorphous (3.2 g, yield 94%).
¹H-NMR (400MHz, DMSO-d₆, TMS): 7.52-7.30 (6H, m),7.09 (1H, s), 7.07 (1H, dd, J=2.0, 8.4 Hz), 6.89 (1H, d, J=8.4 Hz), 5.07 (1H, d, J=12.4 Hz), 5.04 (1H, d, J=12.4 Hz), 4.05 (2H, q, J=6.8 Hz), 3.28 (1H, d, J=13.6 Hz), 2.82 (1H, d, J=13.6 Hz), 1.41 (9H, s), 1.34 (3H, t, J=6.8 Hz), 1.16 (3H, s)

### d) Synthesis of (S)-N-Boc-3-(4,4,5,5-tetramethyl-1,3,2-dioxaboran-2-yl)-O-ethyl-α-methyltyrosine benzyl ester (17) (Compound (17))

Under nitrogen atmosphere, to a mixture of Compound (16) (3.2 g, 5.9 mmol), [1,1'-bis(diphenylphosphino)ferrocene]dichloropalladium(II) dichloromethane adduct (0.37 g, 0.45 mmol), potassium acetate (2.3 g, 23.7 mmol) and dimethyl sulfoxide (27 mL) was added bis(pinacolato)diboron (3.0 g, 11.8 mmol), and the mixture was stirred at room temperature for 1 hr, and then stirred at 55°C for 2 hr. The reaction solution was allowed to cool to room temperature, methyl t-butyl ether (50 mL) and water (50 mL) were added, and the mixture was filtered through Celite, and subjected to liquid separation. The aqueous layer was subjected to extraction with methyl t-butyl ether (30 mL), and the organic layers were combined, and washed with water (30 mL) and saturated brine (30 mL), and the solvent was evaporated under reduced pressure. The residue was purified by silica gel column chromatography to give Compound (17) as a white amorphous (1.8 g, yield 57%). ¹H-NMR (400MHz, CDCl₃, TMS): 7.42-7.30 (6H, m), 7.02 (1H, dd, J=2.4, 8.4 Hz), 6.70 (1H, d, J=8.4 Hz), 5.19 (1H, d, J=12.4 Hz), 5.12 (1H, d, J=12.4 Hz), 3.99 (2H, q, J=6.8 Hz), 3.34-3.12 (2H, m), 1.45 (9H, s), 1.39 (3H, t, J=6.8 Hz), 1.32 (12H, s), 1.26 (3H, s)

### e) Synthesis of (S)-3-(4,4,5,5-tetramethyl-1,3,2-dioxaboran-2-yl)-O-ethyl-α-methyltyrosine hydrochloride (18) (Compound (18) Bpin-AMT-OEt)

A mixture of Compound (17) (1.8 g, 3.3 mmol), 10% palladium-carbon (55% hydrous, 0.45 g) and tetrahydrofuran (18 mL) was stirred under hydrogen gas atmosphere for 3 hr. The reaction solution was filtered through Celite, and the solvent was evaporated under reduced pressure to give a white amorphous (1.4 g). To a mixture of the obtained amorphous (1.4 g, 3.1 mmol) and ethyl acetate (4.2 mL) was added 4M hydrochloric acid-ethyl acetate solution (4.2 mL), and the mixture was stirred at room temperature for 15 hr. The precipitated solid was collected by filtration, and washed with ethyl acetate to give a white powder (0.67 g). The powder was washed by suspension with ethyl acetate (15.0 mL) at room temperature for 1 hr. The solid was collected by filtration, and washed with ethyl acetate to give Compound (18) as a white powder (0.52 g, yield 41%).
¹H-NMR (400MHz, DMSO-d₆, TMS): 8.31 (3H, br), 7.31 (1H, d, J=2.4 Hz), 7.24 (1H, dd, J=2.4, 8.4 Hz), 6.91 (1H, d, J=8.4 Hz), 3.98 (2H, q, J=6.8 Hz), 3.07 (1H, d, J=14.0 Hz), 2.97 (1H, d, J=14.0 Hz), 1.45 (3H, s), 1.29 (3H, t, J=6.8 Hz), 1.27 (12H, s)

### HPLC analysis condition

sample solution: 1 mg of Compound (18) was dissolved in 67% acetonitrile-containing water (1 mL) to prepare a sample solution.
detector: UV 275 nm
column: YMC-Pack Pro C18 RS (4.6 mmφ×25 cm, 5 µm, YMC)
column temperature: 30°C
mobile phase: H₂O (0.1% TFA)/MeCN (0.1% TFA)
mode: gradient
   0 min H₂O (0.1% TFA)/MeCN (0.1% TFA) = 95/5
   30 min H₂O (0.1% TFA)/MeCN (0.1% TFA) = 5/95
   40 min H₂O (0.1% TFA)/MeCN (0.1% TFA) = 5/95
flow rate: 1 mL/min
injection volume: 5 µL
analysis time: 40 min
retention time: Compound (18) 9.4 min

The HPLC chart is shown in Fig.7.

### Example 6

### Synthesis of (S)-3-astato(²¹¹At)-O-ethyl-α-methyltyrosine (²¹¹At-AAMT-OEt)

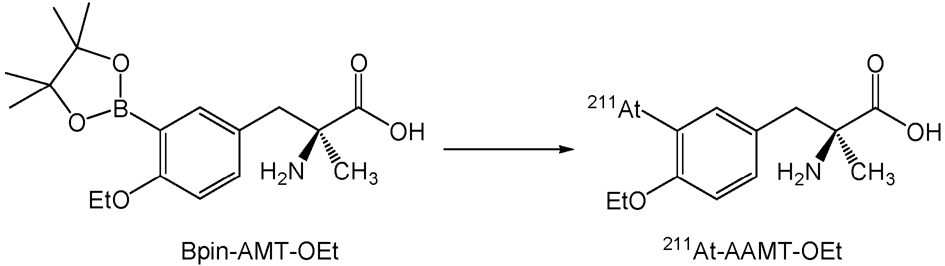

Compound (18) (Bpin-AMT-OEt) was dissolved in Meylon (7%) to prepare an aqueous solution (1 mg/mL). This aqueous solution (0.1 mL) was put in a 1.5 mL polypropylene (PP) tube. Then, the ²¹¹At aqueous solution (100 µL, 5 MBq, the radiation dose was measured using a curie meter (IGC-7, Hitachi)) prepared in Reference Example 1 and 0.1M KI aqueous solution (20 µL) were added, followed by sterile distilled water to bring the total volume to 200 µL. The mixture was reacted at 50°C for 45 min to give the crude product solution of (S)-3-astato (²¹¹At) -O-ethyl-α-methyltyrosine (²¹¹At-AAMT-OEt).

The crude product solution (1 µL) was analyzed by thin-layer chromatography (TLC). The sample was spotted on a thin-layer plate (silica gel 60F₂₅₄), and a mixed solvent of acetonitrile/water (2/1) was used as a developing solvent. The thin-layer plate was exposed to an imaging plate (BAS IP MS 2025E, GE Healthcare) for about 15 min, and then analyzed by a bioimage analyzer (Tyhoon^{™} FLA-7000, Cytiva). ²¹¹At-AAMT-OEt in the crude product solution was detected at Rf 0.80, and the radiochemical yield (RCY) was 85.5%.

The crude product solution was injected into an HLB column (Oasis, WAT-186005125) equilibrated with sterile distilled water (1 mL) to capture the target product in the cartridge, and sterile distilled water was passed through the cartridge to elute impurities. Next, 30% ethanol solution (500 µL) was passed through the cartridge to elute the target product into sodium ascorbate aqueous solution (500 µL, final concentration 1%), thus obtaining the purified product. This purified product was analyzed by thin-layer chromatography (TLC) in the same manner as described above. The results are shown in Fig.8. The radiochemical yield and radiochemical purity of the purified product were 75.9% and 99.27%, respectively.

24 hours after production, ²¹¹At-AAMT-OEt was analyzed by thin-layer chromatography (TLC) in the same manner as described above. The results are shown in Fig.9. As shown in Fig.9, no decomposition of ²¹¹At-AAMT-OEt was observed even 24 hours after purification.

### Example 7

### Synthesis of (S)-3-(4,4,5,5-tetramethyl-1,3,2-dioxaboran-2-yl)-O-propyl-α-methyltyrosine hydrochloride (24) (Compound (24) Bpin-AMT-OPr)

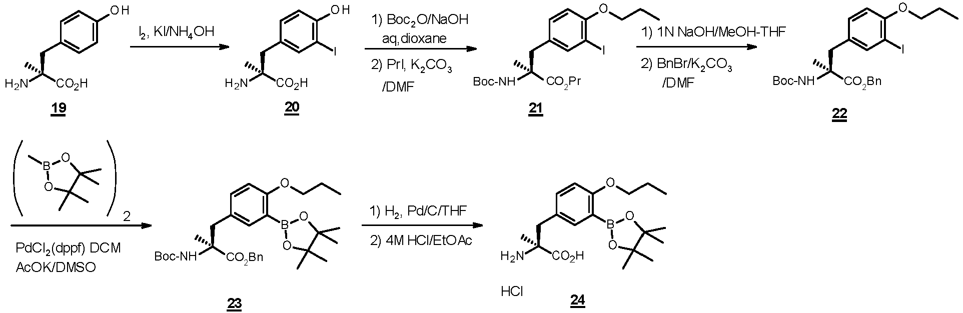

### a) Synthesis of (S)-3-iodo-α-methyltyrosine (20) (Compound (20))

To a mixture of potassium iodide (5.3 g, 32.1 mmol) and water (14 mL) was added iodine (7.1 g, 28.1 mmol), and the mixture was stirred for 2 hr, and added to a mixture (cooled to -7°C) of (S)-α-methyltyrosine (19) (5.0 g, 25.6 mmol), concentrated aqueous ammonia (28%, 62 mL) and water (8 mL) at - 5°C or lower, and the mixture was stirred at -7 to -5°C for 3 hr. To the reaction solution was added 15% aqueous sodium sulfite solution (15 mL), and the mixture was allowed to warm to room temperature, adjusted to pH 6.5 to 7 with 6M hydrochloric acid under ice-cooling, and stirred under ice-cooling for 1 hr. The precipitated solid was collected by filtration, washed with cold water and acetone, and dried under reduced pressure to give Compound (20) as a white solid (8.4 g, yield 98%).
¹H-NMR (400MHz, DMSO-d₆+TFA, TMS): 8.25(3H, br), 7.51 (1H, d, J=2.0 Hz), 7.03 (1H, dd, J=2.0, 8.4 Hz), 6.84 (1H, d, J=8.4 Hz), 3.03 (1H, d, J=14.1 Hz), 2.87 (1H, d, J=14.1 Hz), 1.44

### (3H, s)

### b) Synthesis of (S)-N-Boc-3-iodo-O-propyl-α-methyltyrosine propyl ester (21) (Compound (21))

To a mixture of Compound (20) (4.2 g, 13.1 mmol), 1M aqueous sodium hydroxide solution (30 mL, 2.3 eq.), water (25 mL) and 1,4-dioxane (50 mL) was added di-t-butyl dicarbonate (3.1 g, 14.4 mmol, 1.1 eq.), and the mixture was stirred at 60°C for 22 hr. The reaction solution was allowed to cool to room temperature, and concentrated under reduced pressure. Water (30 mL) and methyl t-butyl ether-heptane (1:2, 60 mL) were added, and the mixture was subjected to liquid separation, and the organic layer was subjected to extraction with 5% aqueous sodium hydrogencarbonate solution (20 mL) and water (20 mL). The combined aqueous layers were adjusted to pH 1-2 with 0.5M aqueous sodium hydrogen sulfate solution, and subjected to extraction with methyl t-butyl ether-ethyl acetate (1:1, 60 mL). The extract was washed with water and saturated brine, and the solvent was evaporated under reduced pressure to give a pale-brown solid (2.1 g). The obtained pale-brown solid (2.1 g, 5.0 mmol) was dissolved in N,N-dimethylformamide (20 mL), potassium carbonate (1.5 g, 11 mmol, 2.2 eq.) and propyl iodide (1.1 mL, 11 mmol, 2.2 eq.) were added, and the mixture was stirred at room temperature for 18 hr. Ethyl acetate (60 mL) was added, the mixture was washed with water (60 mL) and saturated brine (30 mL), and the solvent was evaporated under reduced pressure. The residue was purified by silica gel column chromatography (Yamazen 2L size column, ethyl acetate-heptane = 1/19→1/9→2/8→3/7→5/5) to give Compound (21) as a white amorphous (2.0 g, yield 30%).
¹H-NMR (400MHz, CDCl₃, TMS): 7.50 (1H, d, J=2.0 Hz), 6.98 (1H, dd, J=2.0, 8.4 Hz), 6.68 (1H, d, J=8.4 Hz), 5.18 (1H, brs), 4.18-4.04 (2H, m), 3.33 (1H, d, J=13.6 Hz), 3.11 (1H, d, J=13.6 Hz), 1.88-1.64 (4H, m), 1.56 (3H, s), 1.49 (9H, s), 1.09 (3H, t, J=7.2 Hz), 0.98 (3H, t, J=7.2 Hz)

### c) Synthesis of (S)-N-Boc-3-iodo-O-propyl-α-methyltyrosine benzyl ester (22) (Compound (22))

To a mixture of Compound (21) (3.1 g, 6.1 mmol) in methanol (16 mL) and tetrahydrofuran (16 mL) was added 2M aqueous sodium hydroxide solution (11.0 mL, 22.0 mmol, 3.6 eq.), and the mixture was stirred at 60°C for 17 hr. The reaction solution was allowed to cool to room temperature, and concentrated. To the residue were added water (30 mL) and methyl t-butyl ether (30 mL), and the mixture was subjected to liquid separation. The aqueous layer was adjusted to pH 2-3 with 0.5M aqueous sodium hydrogen sulfate solution, and subjected to extraction with methyl t-butyl ether (30 mL). The extract was washed with water (20 mL), and the solvent was evaporated under reduced pressure to give a white amorphous (2.9 g). The obtained white amorphous (2.9 g) was dissolved in N,N-dimethylformamide (16 mL), potassium carbonate (1.0 g, 7.4 mmol, 1.2 eq.) and benzyl bromide (1.3 mL, 7.4 mmol, 1.2 eq.) were added, and the mixture was stirred at 50°C for 3 hr, and allowed to cool to room temperature. Ethyl acetate (30 mL) was added, and the mixture was washed with water (30 mL) and saturated brine (30 mL), and the solvent was evaporated under reduced pressure. The residue was purified by silica gel column chromatography (Yamazen 2L size column, ethyl acetate-heptane =1/9→2/8→3/7) to give Compound (22) as a white amorphous (3.0 g, yield 89%).
¹H-NMR (400MHz, DMSO-d₆, TMS): 7.44 (1H, brs), 7.38-7.30 (5H, m), 7.08 (1H, brs), 7.07 (1H, dd, J=2.0, 8.4 Hz), 6.89 (1H, d, J=8.4 Hz), 5.11 (1H, d, J=12.4 Hz), 5.03 (1H, d, J=12.4 Hz), 3.95 (2H, t, J=6.4 Hz), 3.28 (1H, d, J=13.2 Hz), 2.72 (1H, d, J=13.2 Hz), 1.77-1.68(2H, m), 1.42 (9H, s), 1.18 (3H, s), 1.02 (3H, t, J=7.2 Hz)

### d) Synthesis of (S)-N-Boc-3-(4,4,5,5-tetramethyl-1,3,2-dioxaboran-2-yl)-O-propyl-α-methyltyrosine benzyl ester (23) (Compound (23))

Under nitrogen atmosphere, to a mixture of Compound (22) (3.0 g, 5.4 mmol), [1,1'-bis(diphenylphosphino)ferrocene]dichloropalladium(II) dichloromethane adduct (0.34 g, 0.41 mmol, 0.076 eq.), potassium acetate (2.1 g, 21.7 mmol, 4 eq.) and dimethyl sulfoxide (22 mL) was added bis(pinacolato)diboron (2.8 g, 10.8 mmol, 2 eq.), and the mixture was stirred at room temperature for 1 hr, and then stirred at 55°C for 2 hr. The reaction solution was allowed to cool to room temperature, methyl t-butyl ether (50 mL) and water (50 mL) were added, and the mixture was filtered through Celite, and subjected to liquid separation. The aqueous layer was subjected to extraction with methyl t-butyl ether (30 mL), and the organic layers were combined, and washed with water (30 mL) and saturated brine (30 mL), and the solvent was evaporated under reduced pressure. The residue was purified by silica gel column chromatography (Yamazen 2L size column, ethyl acetate-heptane =1/19→1/9→2/8→3/7) to give Compound (23) as a white amorphous (1.5 g, yield 50%).
¹H-NMR (400MHz, CDCl₃, TMS): 7.40-7.32 (6H, m), 7.02 (1H, dd, J=2.0, 8.4 Hz), 6.69 (1H, d, J=8.4 Hz), 5.19 (1H, d, J=12.4 Hz), 5.13 (1H, d, J=12.4 Hz), 3.89 (2H, t, J=6.4 Hz), 3.28 (1H, d, J=13.6 Hz), 3.16 (1H, d, J=13.6 Hz), 1.85-1.76(2H, m), 1.57 (3H, s), 1.45 (9H, s), 1.31 (12H, s), 1.07 (3H, t, J=7.2 Hz)

### e) Synthesis of (S)-3-(4,4,5,5-tetramethyl-1,3,2-dioxaboran-2-yl)-O-propyl-α-methyltyrosine hydrochloride (24) (Compound (24)

### Bpin-AMT-OPr)

A mixture of Compound (23) (600 mg, 1.1 mmol), 10% palladium-carbon (55% hydrous, 150 mg) (Pd 1.5 wt%) and tetrahydrofuran (6 mL) was stirred under hydrogen gas atmosphere for 3 hr. The reaction solution was filtered through Celite, and the solvent was evaporated under reduced pressure to give a white amorphous (530 mg). To a mixture of the obtained amorphous (530 mg, 1.1 mmol) and ethyl acetate (4.0 mL) was added 4M hydrochloric acid-ethyl acetate solution (2 mL), and the mixture was stirred at room temperature for 3 hr. The precipitated solid was collected by filtration, and washed with ethyl acetate (5.0 mL) to give Compound (24) as a white powder (0.18 g, yield 43%). ¹H-NMR (400MHz, DMSO-d₆, TMS): 8.32 (3H, br), 7.33 (1H, d, J=2.4 Hz), 7.24 (1H, dd, J=2.4, 8.4 Hz), 6.90 (1H, d, J=8.4 Hz), 3-91-3.85 (2H, m), 3.08 (1H, d, J=14.4 Hz), 2.97 (1H, d, J=14.4 Hz), 1.74-1.66(2H, m), 1.46 (3H, s), 1.27 (12H, s), 1.02 (3H, t, J=7.2 Hz)

### HPLC analysis condition

sample solution: 1 mg of Compound (24) was dissolved in 67% acetonitrile-containing water (1 mL) to prepare a sample solution.
detector: UV 275 nm
column: YMC-Pack Pro C18 RS (4.6 mmφ×25 cm, 5 µm, YMC)
column temperature: 30°C
mobile phase: H₂O (0.1% TFA)/MeCN (0.1% TFA)
mode: gradient
   0 min H₂O (0.1% TFA)/MeCN (0.1% TFA) = 95/5
   30 min H₂O (0.1% TFA)/MeCN (0.1% TFA) = 5/95
   40 min H₂O (0.1% TFA)/MeCN (0.1% TFA) = 5/95
flow rate: 1 mL/min
injection volume: 5 µL
analysis time: 40 min
retention time: Compound (24) 10.6 min

The HPLC chart is shown in Fig.10.

### Example 8

### Synthesis of (S)-3-astato(²¹¹At)-O-propyl-α-methyltyrosine (²¹¹At-AAMT-OPr)

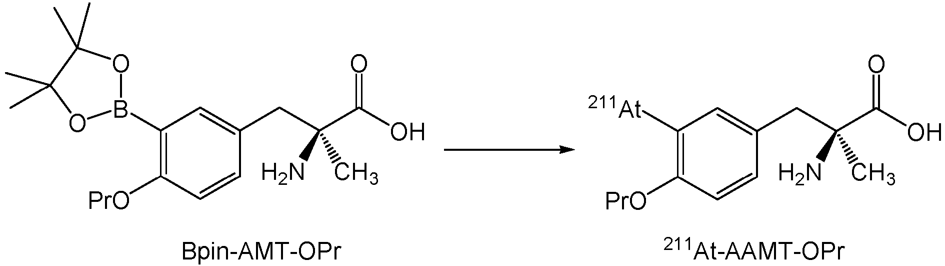

Compound (24) (Bpin-AMT-OPr) was dissolved in Meylon (7%) to prepare an aqueous solution (1 mg/mL). This aqueous solution (0.1 mL) was put in a 1.5 mL polypropylene (PP) tube. Then, the ²¹¹At aqueous solution (100 µL, 20 MBq, the radiation dose was measured using a curie meter (IGC-7, Hitachi)) prepared in Reference Example 1 and 0.1M KI aqueous solution (20 µL) were added, followed by sterile distilled water to bring the total volume to 200 µL. The mixture was reacted at 50°C for 45 min to give the crude product solution of (S)-3-astato (²¹¹At) -O-propyl-α-methyltyrosine (²¹¹At-AAMT-OPr).

The crude product solution (1 µL) was analyzed by thin-layer chromatography (TLC). The sample was spotted on a thin-layer plate (silica gel 60F₂₅₄), and a mixed solvent of acetonitrile/water (2/1) was used as a developing solvent. The thin-layer plate was exposed to an imaging plate (BAS IP MS 2025E, GE Healthcare) for about 15 min, and then analyzed by a bioimage analyzer (Tyhoon^{™} FLA-7000, Cytiva). ²¹¹At-AAMT-OPr in the crude product solution was detected at Rf 0.80, and the radiochemical yield (RCY) was 78.0%.

The crude product solution was injected into an HLB column (Oasis, WAT-186005125) equilibrated with sterile distilled water (1 mL) to capture the target product in the cartridge, and sterile distilled water was passed through the cartridge to elute impurities. Next, 30% ethanol solution (500 µL) was passed through the cartridge to elute the target product into sodium ascorbate aqueous solution (500 µL, final concentration 1%), thus obtaining the purified product. This purified product was analyzed by thin-layer chromatography (TLC) in the same manner as described above. The results are shown in Fig.11. The radiochemical yield and radiochemical purity of the purified product were 78.0% and 99.52%, respectively.

24 hours after production, ²¹¹At-AAMT-OPr was analyzed by thin-layer chromatography (TLC) in the same manner as described above. The results are shown in Fig.12. As shown in Fig.12, no decomposition of ²¹¹At-AAMT-OPr was observed even 24 hours after purification.

### Example 9

### Synthesis of (S)-α-methyl-4-(4,4,5,5-tetramethyl-1,3,2-dioxaboran-2-yl)-3-methoxyphenylalanine hydrochloride (28) (Compound (28) Bpin-AMPhe-OMe)

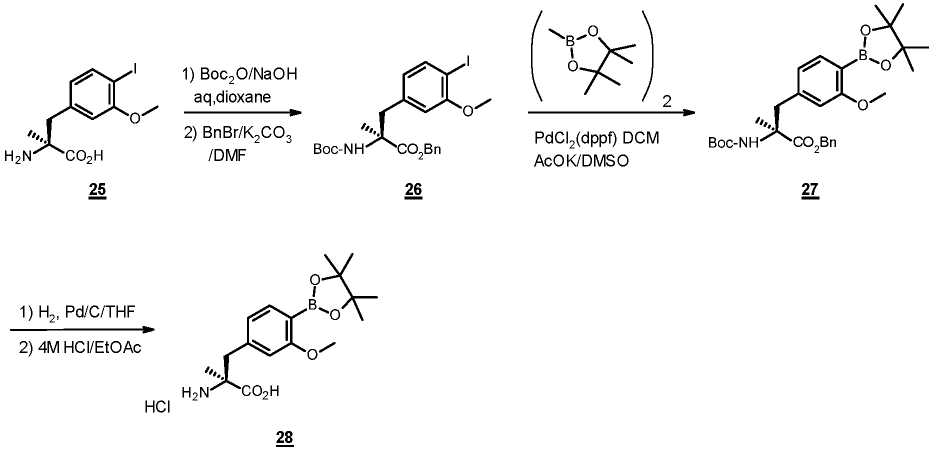

### a) Synthesis of (S)-N-Boc-α-methyl-4-iodo-3-methoxyphenylalanine benzyl ester (26) (Compound (26))

To a mixture of (S)-α-methyl-4-iodo-3-methoxyphenylalanine Compound (25) (1.3 g, 3.9 mmol), 1M aqueous sodium hydroxide solution (8.9 mL, 2.3 eq.), water (6.5 mL) and 1,4-dioxane (13 mL) was added di-t-butyl dicarbonate (930 mg, 4.3 mmol, 1.1 eq.), and the mixture was stirred at 60°C for 22 hr. The reaction solution was allowed to cool to room temperature, and concentrated under reduced pressure. Water (30 mL) and methyl t-butyl ether-heptane (1:2, 60 mL) were added, and the mixture was subjected to liquid separation, and the organic layer was subjected to extraction with 5% aqueous sodium hydrogencarbonate solution (20 mL) and water (20 mL). The combined aqueous layers were adjusted to pH 1-2 with 0.5M aqueous sodium hydrogen sulfate solution, and subjected to extraction with methyl t-butyl ether-ethyl acetate (1:1, 50 mL). The extract was washed with water and saturated brine, and the solvent was evaporated under reduced pressure to give a white amorphous (1.3 g). The obtained white amorphous (1.3 g) was dissolved in N,N-dimethylformamide (13 mL), potassium carbonate (0.7 g, 5.1 mmol, 1.2 eq.) and benzyl bromide (0.58 mL, 5.1 mmol, 1.2 eq.) were added, and the mixture was stirred at 50°C for 3 hr, and allowed to cool to room temperature. Ethyl acetate (30 mL) was added, and the mixture was washed with water (30 mL) and saturated brine (30 mL), and the solvent was evaporated under reduced pressure. The residue was purified by silica gel column chromatography (Yamazen 2L size column, ethyl acetate-heptane =1/19→1/9→2/8) to give Compound (26) as a white amorphous (1.1 g, yield 54%).
¹H-NMR (400MHz, CDCl₃, TMS): 7.57 (1H, d, J=7.6 Hz), 7.41-7.31 (5H, m), 6.53 (1H, d, J=1.6 Hz), 6.36 (1H, dd, J=1.6, 7.6 Hz), 5.22-5.13 (3H, m), 3.77 (3H, s), 3.36 (1H, d, J=13.6 Hz), 3.23 (1H, d, J=13.2 Hz), 1.59 (3H, s), 1.44 (9H, s)

### b) Synthesis of (S)-N-Boc-4-(4,4,5,5-tetramethyl-1,3,2-dioxaboran-2-yl)-α-methyl-3-methoxyphenylalanine benzyl ester (27) (Compound (27))

Under nitrogen atmosphere, to a mixture of Compound (26) (1.1 g, 2.1 mmol), [1,1'-bis(diphenylphosphino)ferrocene]dichloropalladium(II) dichloromethane adduct (0.13g, 0.16 mmol, 0.076 eq.), potassium acetate (0.82 g, 8.4 mmol, 4 eq.) and dimethyl sulfoxide (8.4 mL) was added bis(pinacolato)diboron (1.1 g, 4.2 mmol, 2 eq.), and the mixture was stirred at room temperature for 1 hr, and then stirred at 55°C for 1 hr. The reaction solution was allowed to cool to room temperature, methyl t-butyl ether (30 mL) and water (30 mL) were added, and the mixture was filtered through Celite, and subjected to liquid separation. The aqueous layer was subjected to extraction with methyl t-butyl ether (30 mL), and the organic layers were combined, and washed with water (30 mL) and saturated brine (30 mL), and the solvent was evaporated under reduced pressure. The residue was purified by silica gel column chromatography (Yamazen 2L size column, ethyl acetate-heptane =1/19→1/9→2/8) to give Compound (27) as a white amorphous (0.61 g, yield 56%).
¹H-NMR (400MHz, CDCl₃, TMS): 7.54 (1H, d, J=7.2 Hz), 7.40-7.31 (5H, m), 6.64 (1H, dd, J=1.2, 7.2 Hz), 6.58 (1H, d, J=1.2 Hz), 5.22-5.06 (3H, m), 3.76 (3H, s), 3.40-3.21 (2H, m), 1.63 (3H, s), 1.43 (9H, s)

### c) Synthesis of (S)-α-methyl-4-(4,4,5,5-tetramethyl-1,3,2-dioxaboran-2-yl)-3-methoxyphenylalanine hydrochloride (28) (Compound (28) Bpin-AMPhe-OMe)

A mixture of Compound (27) (600 mg, 1.1 mmol), 10% palladium-carbon (55% hydrous, 150 mg) (Pd 1.5 wt%) and tetrahydrofuran (6 mL) was stirred under hydrogen gas atmosphere for 3 hr. The reaction solution was filtered through Celite, and the solvent was evaporated under reduced pressure to give a white amorphous (480 mg). To a mixture of the obtained amorphous (480 mg, 1.1 mmol) and ethyl acetate (4.0 mL) was added 4M hydrochloric acid-ethyl acetate solution (2 mL), and the mixture was stirred at room temperature for 24 hr. The reaction solution was concentrated, and MTBE (10 mL) was added, followed by heptane (10 mL). The precipitated solid was collected by filtration, and washed with a mixed solvent (1/1, 5.0 mL) of MTBE/heptane to give Compound (28) as a white powder (0.14 g, yield 33%).
¹H-NMR (400MHz, D₂O): 7.61 (1H, d, J=12.8 Hz), 6.91 (2H, s), 3.91 (3H, s), 3.36 (1H, d, J=14.0 Hz), 3.04 (1H, d, J=14.0 Hz), 1.60 (3H, s), 1.21 (12H, s)

### HPLC analysis condition

sample solution: 1 mg of Compound (28) is dissolved in 67% acetonitrile-containing water (1 mL) to prepare a sample solution.
detector: UV 275 nm
column: YMC-Pack Pro C18 RS (4.6 mmφ×25 cm, 5 µm, YMC)
column temperature: 30°C
mobile phase: H₂O (0.1% TFA)/MeCN (0.1% TFA)
mode: gradient
   0 min H₂O (0.1% TFA)/MeCN (0.1% TFA) = 95/5
   30 min H₂O (0.1% TFA)/MeCN (0.1% TFA) = 5/95
   40 min H₂O (0.1% TFA)/MeCN (0.1% TFA) = 5/95
flow rate: 1 mL/min
injection volume: 5 µL
analysis time: 40 min
retention time: Compound (28) 7.4 min

The HPLC chart is shown in Fig.13.

### Example 10

### Synthesis of (S)-α-methyl-4-astato(²¹¹At)-3-methoxyphenylalanine (²¹¹At-AAMPhe-OMe)

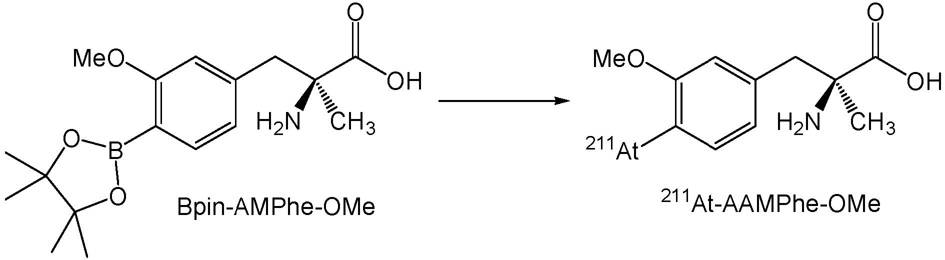

A crude product solution of (S)-α-methyl-4-astato(²¹¹At)-3-methoxyphenylalanine (²¹¹At-AAMPhe-OMe) was obtained from Compound (28) (Bpin-AMPhe-OMe) by a method similar to that described in Example 2 (the amount of ²¹¹At use was 1.525 MBq).

The crude product solution was analyzed by thin-layer chromatography (TLC) by a method similar to that described in Example 2. ²¹¹At-AAMPhe-OMe in the crude product solution was detected at Rf 0.80, and the radiochemical yield (RCY) was 61.34%.

The crude product solution was sterilized and treated with sodium ascorbate aqueous solution by a method similar to that described in Example 2 to give the purified product. This purified product was analyzed by thin-layer chromatography (TLC) by a method similar to that described in Example 2. The results are shown in Fig.14. The radiochemical yield and radiochemical purity of the purified product were 65.11% and 97.91%, respectively.

24 hours after production, ²¹¹At-AAMPhe-OMe was analyzed by thin-layer chromatography (TLC) in the same manner as described above. The results are shown in Fig.15. As shown in Fig.15, no decomposition of ²¹¹At-AAMPhe-OMe was observed even 24 hours after purification.

Bpin-AMPhe-OEt and Bpin-AMPhe-OPr, which are the corresponding boronic acid pinacol ester, can be synthesized by a method similar to that described in Example 9 or a method analogous thereto, and ²¹¹At-AAMPhe-OEt and ²¹¹At-AAMPhe-OPr, which are the corresponding 4-²¹¹At form, can be synthesized from these compounds by a method similar to that described in Example 2 or a method analogous thereto.

### Test Example 1 Cellular uptake test (using HEK293)

### <Culture of cells>

Cells to be used: HEK293-Mock, HEK293-hLAT1, HEK293-hLAT2 (HEK293: Human Embryonic Kidney cells 293)
Culture medium to be used: E-MEM (containing 10% FBS, 1% P/S, 1% NEAA)

Two days before a test day, a cell suspension was prepared in such a manner that the concentration of predetermined cells was 1 x 10⁵ cells/mL. The cell suspension was seeded in a 24-well multiculture plate at a volume of 0.5 mL per well. On the day of the test, cultures were confirmed to be subconfluent (which refers to a state where 70-80% of the well is covered in density). The cells were washed twice with warmed PBS(-) Buffer (166-23555, FUJIFILM Wako Pure Chemical Corporation), the culture medium was replaced with HBSS(-) Buffer (085-09355, FUJIFILM Wako Pure Chemical Corporation), and the plate was left to stand in an incubator (MINIcell-35, WakenBtech Co., Ltd) until an RI addition test.

### <Uptake test into cultured cells>

²¹¹At-AAMT-OMe or ²¹¹At-AAMT was added to the prepared cells at 10 µL/well. BCH (LAT1 inhibitor, 20 mM) or unlabeled AMT (α-methyl tyrosine, 1 µg/mL) was previously (15 minutes before) added (10 µg/mL). The cells were cultured (CO₂ 5%, 37°C), and after 30 minutes, the liquid was removed by suction using an aspirator. The cells were washed twice with PBS(-). The cells were lysed by adding 0.1N NaOH to obtain a lysate, and the radioactivity of the lysate was measured using a γ counter. After the radioactivity measurement, the lysate was loaded into a 96-well plate at a volume of 10 µL per well, the protein amount was measured by the BCA method, and the number of cells was corrected based on the protein amount. Absorbance was measured by MultiScanFC (Thermo Fisher). The uptake level of ²¹¹At-AAMT-OMe or ²¹¹At-AAMT into the cells (number of radioactivity counts/protein amount) was calculated. The results are shown in Fig. 16.

It should be noted that in Fig. 16, CTL is control, BCH is 2-aminobicyclo[2,2,1]heptane-2-carboxylic acid, and AMT is α-methyl tyrosine. As can be seen from Fig. 16, ²¹¹At-AAMT-OMe was strongly taken up into HEK293-hLAT1 cells (CTL), comparable to ²¹¹At-AAMT, and its uptake was significantly inhibited in the presence of BCH and AMT. These results confirmed that ²¹¹At-AAMT-OMe was specifically taken up into HEK293-hLAT1 cells.

### Test Example 2 Imaging of mice subcutaneously transplanted with human pancreatic cancer cell line, PANC1

Immunodeficient mice (Balb/c-nu/nu, 5-week-old, male) were habituated for 1 week and then subcutaneously transplanted with a human pancreatic cancer cell line (PANC1, 1 × 10⁷ cells/mouse). After the transplantation, the mice were observed over time until tumor reached a size large enough for imaging (about 1000 mm³). When the tumor reached a required size, ²¹¹At-AAMT-OMe or ²¹¹At-AAMT diluted with water for injection to 0.5 MBq/0.2 mL per mouse was administered via tail vein. Two hours after administration, the mice were anesthetized by isoflurane inhalation, and planar images were taken using a γ camera (E-cam, Siemens). The results are shown in Fig. 17.

As can be seen from Fig. 17, ²¹¹At-AAMT-OMe and ²¹¹At-AAMT are both accumulated in the tumor sites, with a larger amount of ²¹¹At-AAMT-OMe remaining in the tumor.

### Test Example 3 Therapeutic test on mice subcutaneously transplanted with human pancreatic cancer cell line, PANC1

In the same manner as in Test Example 2, mice were subcutaneously transplanted with a human pancreatic cancer cell line (PANC1, 1 × 10⁷ cells/mouse). The mice were observed over time until engraftment of tumors was confirmed (50 to 100 mm³), and when the tumors reached a predetermined size, ²¹¹At-AAMT-OMe or ²¹¹At-AAMT diluted with water for injection to 1 MBq/0.2 mL per mouse was administered via tail vein. Twenty-eight days after administration, the mice were euthanized and subjected to autopsy, and tumor masses were isolated after organs were examined for abnormalities. After fat tissue and the like were trimmed off, the wet weights of the isolated tumor masses were measured to make a histogram. The results are shown in Fig. 18.

As can be seen from Fig. 18, the tumor size of the ²¹¹At-AAMT-OMe administration group was slightly smaller than that of the ²¹¹At-AAMT administration group. This confirms that the anti-tumor effect of ²¹¹At-AAMT-OMe is slightly higher than that of ²¹¹At-AAMT.

Further, changes in mouse body weight from immediately after administration to 28th day after administration are shown in Fig. 19. As can be seen from Fig. 19, the mouse body weight of the ²¹¹At-AAMT-OMe administration group tends to slightly reduce, but the body weight reduction is significantly smaller than that of the control group, and is therefore not at a level regarded as a side effect, considering the increase in tumor size.

### Test Example 4 Cellular uptake test (using PANC1)

### <Culture of cells>

Cells to be used: PANC1
Culture medium to be used: D-MEM (containing 10% FBS, 1% P/S)

Two days before a test day, a cell suspension was prepared in such a manner that the concentration of predetermined cells was 1 x 10⁵ cells/mL in the same manner as in Test Example 1. The cell suspension was seeded in a 24-well multiculture plate at a volume of 0.5 mL per well. On the day of the test, cultures were confirmed to be subconfluent (which refers to a state where 70-80% of the well is covered in density). The cells were washed twice with warmed PBS(-) Buffer (166-23555, FUJIFILM Wako Pure Chemical Corporation), the culture medium was replaced with HBSS(-) Buffer (085-09355, FUJIFILM Wako Pure Chemical Corporation), and the plate was left to stand in an incubator (MINIcell-35, WakenBtech Co., Ltd) until an RI addition test.

### <Uptake test into cultured cells>

²¹¹At-AAMT-OMe, ²¹¹At-AAMT-OEt, ²¹¹At-AAMT-OPr, or ²¹¹At-AAMT was added to the prepared cells at 10 µL/well. BCH (LAT1 inhibitor, 20 mM) or unlabeled AMT (α-methyl tyrosine, 1 µg/mL) was previously (15 minutes before) added (10 µg/mL). The cells were cultured (CO₂ 5%, 37°C), and after 30 minutes, the liquid was removed by suction using an aspirator. The cells were washed twice with PBS(-). The cells were lysed by adding 0.1N NaOH to obtain a lysate, and the radioactivity of the lysate was measured using a γ counter. After the radioactivity measurement, the lysate was loaded into a 96-well plate at a volume of 10 µL per well, the protein amount was measured by the BCA method, and the number of cells was corrected based on the protein amount. Absorbance was measured by MultiScanFC (Thermo Fisher). The uptake level of ²¹¹At-AAMT-OMe, ²¹¹At-AAMT-OEt, ²¹¹-At-AAMT-OPr, or ²¹¹At-AAMT into the cells (number of radioactivity counts/protein amount) was calculated.

The results are shown in Fig. 20. It should be noted that in Fig. 20, "OMe" is ²¹¹At-AAMT-OMe, "OEt" is ²¹¹At-AAMT-OEt, "OPr" is ²¹¹At-AAMT-OPr, and "OH" is ²¹¹At-AAMT.

As can be seen from Fig. 20, ²¹¹At-AAMT-OMe is much superior in uptake level to ²¹¹At-AAMT and is much superior also in uptake speed because its uptake level immediately after addition is larger. Also, it can be seen that ²¹¹At-AAMT-OEt and ²¹¹At-AAMT-OPr are also comparable or superior in uptake level and uptake speed to ²¹¹At-AAMT. ²¹¹At is a short-lived nuclide, and therefore it can be said that large uptake into cancer cells in a short period of time is a great advantage.

### Test Example 5 Measurement of accumulated amounts of ²¹¹At-labeled compounds in mice subcutaneously transplanted with human pancreatic cancer cell line, PANC1

Immunodeficient mice (Balb/c-nu/nu, 5-week-old, male) were habituated for 1 week and then subcutaneously transplanted with a human pancreatic cancer cell line (PANC1, 1 × 10⁷ cells/mouse). After the transplantation, the mice were observed over time until tumors reached an adequate size (about 1000 mm³). When the tumors reached a required size, a ²¹¹At-labeled compound diluted with water for injection to 0.1 MBq/0.2 mL per mouse was administered via tail vein. Radiation doses before and after the administration were measured, and a difference between them was defined as an injected dose (ID). Ten minutes and one hour after administration, the mice were anesthetized by isoflurane inhalation, the tumors were isolated by autopsy in chronological order, and their radiation doses and weights were measured using a gamma counter (2480 Wizard², PerkinElmer) and a semimicro scale (Shimadzu Corporation, readability 10 µg), respectively. For comparison between accumulated amounts, accumulated amounts per unit amount (% ID/g) were calculated. The results are shown in Table 1.

In Table 1, the amounts of the ²¹¹At-labeled compounds accumulated in tumors of the PANC1 tumor-bearing models (%ID/g) are shown. It should be noted that in Table 1, "OMe" is ²¹¹At-AAMT-OMe, "OEt" is ²¹¹At-AAMT-OEt, "OPr" is ²¹¹At-AAMT-OPr, and "OH" is ²¹¹At-AAMT.

**[Table 1]**

| Comparison of tumor accumulation of ²¹¹At-labeled compounds in PANC1 tumor-bearing models (%ID/g) | | | | |
|---|---|---|---|---|
| ²¹¹At-labeled compound | OH | OMe | OEt | OPr |
| 10 minutes | 0.29 | 2.59 | 4.21 | 5.91 |
| 1 hour | 2.49 | 3.78 | 3.59 | 9.45 |

As can be seen from Table 1, ²¹¹At-AAMT-OMe, ²¹¹At-AAMT-OEt, and ²¹¹At-AAMT-OPr were all more highly accumulated in the tumor sites than ²¹¹At-AAMT, and among them, ²¹¹At-AAMT-OPr was most highly accumulated 1 hour after administration. This demonstrates that ²¹¹At-AAMT-OMe, ²¹¹At-AAMT-OEt, and ²¹¹At-AAMT-OPr, especially ²¹¹At-AAMT-OPr, have superior retention at tumor sites compared to ²¹¹At-AAMT.

Also, the test was performed using ²¹¹At-AAMPhe-OMe in the same manner as described above, and as a result, its amounts accumulated in tumor 10 minutes and 1 hour after administration were respectively 3.77% ID/g and 6.38% ID/g, which confirms that ²¹¹At-AAMPhe-OMe was more highly accumulated in the tumor sites than ²¹¹At-AAMT.

### Test Example 6 Therapeutic test on mice subcutaneously transplanted with human pancreatic cancer cell line, PANC1

In the same manner as in Test Example 5, mice were subcutaneously transplanted with a human pancreatic cancer cell line (PANC1, 1 × 10⁷ cells/per mouse). The mice were observed over time until engraftment of tumors was confirmed (50 to 100 mm³), and when the tumors reached a predetermined size, ²¹¹At-AAMT-OMe or ²¹¹At-AAMT-OEt diluted with water for injection to 1 MBq/0.2 mL per mouse was administered via tail vein. Tumor sizes and body weights were measured over time after the administration. The experiment was terminated 21 days after administration. Changes in subcutaneous tumor size are shown in Fig. 21. Also, changes in mouse body weight from immediately after administration to 21 days after administration are shown in Fig. 22. It should be noted that in Fig. 21 and Fig. 22, "OMe" is ²¹¹At-AAMT-OMe and "OEt" is ²¹¹At-AAMT-OEt. As for the control, changes in subcutaneous tumor size and body weight of the mice 7 days and thereafter after administration are not shown in Fig. 21 and Fig. 22 because the tumor sizes exceeded 10% of the body weights and the mice reached a humane endpoint.

Fig. 21 demonstrates that the tumor growth inhibiting effect of the ²¹¹At-AAMT-OEt administration group was comparable to that of the ²¹¹At-AAMT-OMe administration group. Also, as a result of autopsy, lesions other than tumor were not observed. A tumor of 1000 mm³ is equivalent to approximately 1 g in mass. From Fig. 22, it can be assumed that the body weight of the control group was sharply reduced, but the body weights of the ²¹¹At-AAMT-OMe administration group and the ²¹¹At-AAMT-OEt administration group were both gently increased, and therefore apparent toxicity induced by their administration was not observed.

### Test Example 7 Establishment of CDDP-resistant non-small cell lung cancer cell line, A549 (A549 CDDP-r)

Cisplatin (CDDP) was added to a human lung cancer cell line, A549 at an initial concentration of 1 µg/mL. After 3 days, dead cells were removed, and CDDP was again added. Cells that grew like A549 even in the presence of CDDP were isolated and maintained for 3 months while gradually increasing the concentration of CDDP in a culture medium. The cell viability of CDDP-resistant A549 (A549 CDDP-r) is shown in Fig. 23.

As can be seen from Fig. 23, CDDP-resistant A549 (A549 CDDP-r) is still alive even at 100 µg/mL. The cells generated by the above procedure were used as CDDP-resistant A549 (A549 CDDP-r) in Test Examples 8 to 10.

### Test Example 8 Cytotoxicity test on CDDP-resistant non-small cell lung cancer cell line, A549 (A549 CDDP-r)

Two days before experiment, A549 and CDDP-resistant A549 (A549 CDDP-r) were each seeded in a 96-well plate at a concentration of 1 × 10⁵ cells/mL. ²¹¹At-AAMT-OMe or ²¹¹At-AAMT adjusted to a predetermined concentration was added to A549 cells and CDDP-resistant A549 cells, respectively, and the cells were cultured (CO₂ 5%, 37°C). After the cultivation for 48 hours, Cell counting kit-8 (DOJINDO LABORATORIES) was added. After the addition, the cells were cultured at 37°C for 1 hour, and cell viability was measured using absorbance at 450 nm as an indicator. The results are shown in Fig. 24.

As can be seen from Fig. 24, ²¹¹At-AAMT has cytotoxicity against CDDP-resistant A549 (A549 CDDP-r), but ²¹¹At-AAMT-OMe has more remarkable cytotoxicity against A549 CDDP-r.

### Test Example 9 Therapeutic test on mice subcutaneously transplanted with CDDP-resistant non-small cell lung cancer cell line, A549 (A549 CDDP-r)

Immunodeficient mice (Balb/c-nu/nu, 5-week-old, male) were habituated for 1 week and then subcutaneously transplanted with A549 or CDDP-resistant A549 (A549 CDDP-r) (1 × 10⁷ cells/mouse). The mice were observed over time until engraftment of tumors was confirmed (50 to 100 mm³), and when the tumors reached a predetermined size, ²¹¹At-AAMT-OMe or ²¹¹At-AAMT diluted with water for injection to 0.5 MBq/0.2 mL per mouse was administered via tail vein. Thirty days after administration, the mice were euthanized and tumor masses were isolated. After fat tissue and the like were trimmed off, the wet weights of the isolated tumor masses were measured to make a histogram. The results are shown in Fig. 25.

As can be seen from Fig. 25, the anti-tumor effect of ²¹¹At-AAMT-OMe on A549 was comparable to that of ²¹¹At-AAMT on A549, but the anti-tumor effect of ²¹¹At-AAMT-OMe on CDDP-resistant A549 (A549 CDDP-r) was higher than that of ²¹¹At-AAMT on CDDP-resistant A549.

### Test Example 10 Expression of LAT1 on cell surface of CDDP-resistant non-small cell lung cancer cell line, A549 (A549 CDDP-r)

A549 and CDDP-resistant A549 (A549 CDDP-r) cells were dispersed in trypsin-EDTA (FUJIFILM Wako Pure Chemical Corporation). The cells were dyed using an antibody against LAT1 (NOV-NBP2-50465AF647, NOVUS Biologicals) in 0.5% BSA/PBS, and the expression levels of LAT1 on the surface of the A549 and CDDP-resistant A549 (A549 CDDP-r) cells were detected using a flow cytometer (Attune NxT, Thermo Fisher Scientific). The results are shown in Fig. 26.

As can be seen from Fig. 26, the expression level of LAT1 on CDDP-resistant A549 (A549 CDDP-r) cells was increased. This demonstrates that there is a relationship between acquisition of anti-cancer drug resistance and high expression of LAT1.

### Test Example 11 Establishment of gemcitabine-resistant human pancreatic cancer cell line, PANC1 (PANC1 Gem-r)

Gemcitabine (Gem) was added to a human pancreatic cancer cell line, PANC1 at an initial concentration of 100 ng/mL. After 3 days, dead cells were removed, and gemcitabine was again added. Cells that grew like a human pancreatic cancer cell line, PANC1 even in the presence of gemcitabine were isolated and maintained for 3 months while gradually increasing the concentration of gemcitabine in a culture medium. The cell viability of a gemcitabine-resistant human pancreatic cancer cell line, PANC1 (PANC1 Gem-r) is shown in Fig. 27.

As can be seen from Fig. 27, the gemcitabine-resistant human pancreatic cancer cell line, PANC1 (PANC1 Gem-r) is still alive even at 30 µg/mL. The cells generated by this procedure were used as a gemcitabine-resistant human pancreatic cancer cell line, PANC1 (PANC1 Gem-r) in Test Example 12.

### Test Example 12 Therapeutic test on mice subcutaneously transplanted with gemcitabine-resistant human pancreatic cancer cell line, PANC1 (PANC1 Gem-r)

Immunodeficient mice (Balb/c-nu/nu, 5-week-old, male) were habituated for 1 week and then subcutaneously transplanted with PANC1 or Gem-resistant PANC1 (PANC1 Gem-r) (1 × 10⁷ cells/mouse). The mice were observed over time until engraftment of tumors was confirmed (50 to 100 mm³), and when the tumors reached a predetermined size, ²¹¹At-AAMT-OMe diluted with water for injection to 1 MBq/0.2 mL per mouse was administered via tail vein. Thirty days after administration, the mice were euthanized and tumor masses were isolated. After fat tissue and the like were trimmed off, the wet weights of the isolated tumor masses were measured to make a histogram. The results are shown in Fig. 28.

As can be seen from Fig. 28, the anti-tumor effect of ²¹¹At-AAMT-OMe is higher on Gem-resistant PANC1 (PANC1 Gem-r) than on PANC1 that is a parental cell line.

Further, changes in tumor size and body weight of the mice from immediately after administration to 14 days after administration are shown in Fig. 29 and Fig. 30, respectively. It should be noted that as for the PANC1-control group, changes in tumor size and body weight of the mice 7 days and thereafter after administration are not shown in Fig. 29 and Fig. 30 because the tumor sizes exceeded roughly 10% of the body weights (A tumor of 1000 mm³ is equivalent to 1 g in mass, and therefore a mouse with a body weight of about 20 g is euthanized at a humane endpoint when the tumor size is significantly more than 2000 mm³). For the same reason, changes in subcutaneous tumor size and body weight of the mice of the PANC1-administration group (1 MBq) 9 days and thereafter after administration are not shown in Fig. 29 and Fig. 30, either.

Fig. 29 and Fig. 30 demonstrate that ²¹¹At-AAMT-OMe has a higher tumor growth inhibiting effect on the gemcitabine-resistant PANC1 cells.

### [Industrial Applicability]

According to the present invention, it is possible to provide compounds that have excellent LAT1 selectivity, have higher retention at tumor or cancer sites, and have a level of clearance that does not cause side effects, and therefore that can exhibit improved anti-tumor effects. It is also possible to provide compounds that can exhibit excellent anti-tumor effects even against tumors or cancers that have acquired resistance to anti-cancer drugs.

Furthermore, the compounds are produced via tyrosine derivatives (Boronic Acid Compound (III) and Boronic Acid Compound (II)), into which a boryl group (-B(OH)₂) or its ester group is introduced, and therefore, the compounds can be produced stably with good purity by a safe method suitable for industrial production of pharmaceuticals, without using any hazardous substances.

This application is based on patent application No. 2022-150608 filed on September 21, 2022 in Japan, the contents of which are encompassed in full herein.

## Claims

1. A radiolabeled compound represented by Formula (I) or a pharmaceutically acceptable salt thereof: 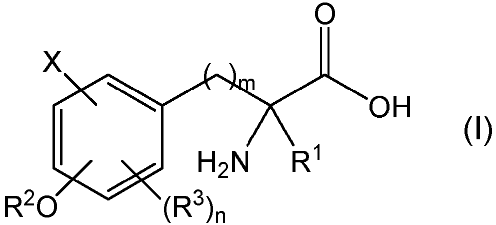 wherein
R¹ is a C₁₋₄ alkyl group;
R² is a C₁₋₆ alkyl group, a C₁₋₆ haloalkyl group or a C₇₋₁₄ aralkyl group;
R³ is a fluorine atom or a chlorine atom;
m is 0, 1 or 2;
n is 0, 1 or 2; and
X is a radionuclide selected from ²¹¹At, ²¹⁰At, ¹³¹I, ¹²⁵I, and ⁷⁷Br.

2. The compound or a pharmaceutically acceptable salt thereof according to Claim 1, wherein m is 1.

3. The compound or a pharmaceutically acceptable salt thereof according to Claim 1, wherein n is 0.

4. The compound or a pharmaceutically acceptable salt thereof according to Claim 1, wherein R² is a C₁₋₆ alkyl group.

5. The compound or a pharmaceutically acceptable salt thereof according to Claim 1, wherein R¹ is a methyl group.

6. The compound or a pharmaceutically acceptable salt thereof according to Claim 1, wherein R²O is bonded to the 4-position on the benzene ring.

7. The compound or a pharmaceutically acceptable salt thereof according to Claim 1, wherein X is bonded to the 3-position on the benzene ring.

8. The compound or a pharmaceutically acceptable salt thereof according to Claim 1, wherein X is ²¹¹At.

9. A pharmaceutical composition comprising the compound or a pharmaceutically acceptable salt thereof as defined in any one of Claims 1 to 8, and a pharmaceutically acceptable carrier.

10. The pharmaceutical composition according to Claim 9, further comprising at least selected from the group consisting of ascorbic acid, an alkali metal ascorbate and an alkalineearth metal ascorbate.

11. A therapeutic agent for a tumor or cancer expressing amino acid transporter LAT1, comprising the compound or a pharmaceutically acceptable salt thereof as defined in any one of Claims 1 to 8.

12. The therapeutic agent according to Claim 11, wherein the tumor or cancer expressing amino acid transporter LAT1 is selected from the group consisting of pancreatic cancer, leukemia, melanoma, colon cancer, lung cancer, prostate cancer, stomach cancer, breast cancer, kidney cancer, laryngeal cancer, esophageal cancer, liver cancer, lymphoma, myeloma, head and neck cancer, ovarian cancer, bladder cancer, childhood cancer, childhood leukemia, brain tumor, osteosarcoma, soft tissue sarcoma, and soft tissue tumor.

13. The therapeutic agent according to Claim 11, wherein the tumor or cancer is a tumor or cancer that has acquired resistance to an anti-cancer drug.

14. The therapeutic agent according to Claim 11, wherein the tumor or cancer is a tumor or cancer that has acquired resistance to a platinum preparation.

15. The therapeutic agent according to Claim 11, wherein the tumor or cancer is a tumor or cancer that has acquired resistance to Cisplatin.

16. The therapeutic agent according to Claim 11, wherein the tumor or cancer is a tumor or cancer that has acquired resistance to a nucleic acid synthesis inhibitor.

17. The therapeutic agent according to Claim 11, wherein the tumor or cancer is a tumor or cancer that has acquired resistance to Gemcitabine.

18. A compound represented by Formula (II) or a salt thereof: 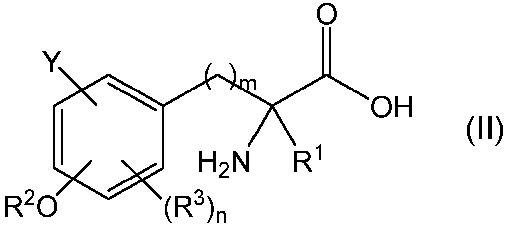 wherein
R¹ is a C₁₋₄ alkyl group;
R² is a C₁₋₆ alkyl group, a C₁₋₆ haloalkyl group or a C₇₋₁₄ aralkyl group;
R³ is a fluorine atom or a chlorine atom;
m is 0, 1 or 2;
n is 0, 1 or 2; and
Y is a boryl group (-B(OH)₂) or its ester group.

19. The compound or a salt thereof according to Claim 18, wherein Y is a boryl group (-B(OH)₂) or a 4,4,5,5-tetramethyl-1,3,2-dioxaboran-2-yl group.

20. An agent for boron neutron capture therapy, comprising the compound or a pharmaceutically acceptable salt thereof as defined in Claim 18 or 19.

21. A method for producing a radiolabeled compound represented by Formula (I) or a pharmaceutically acceptable salt thereof, comprising the following step; 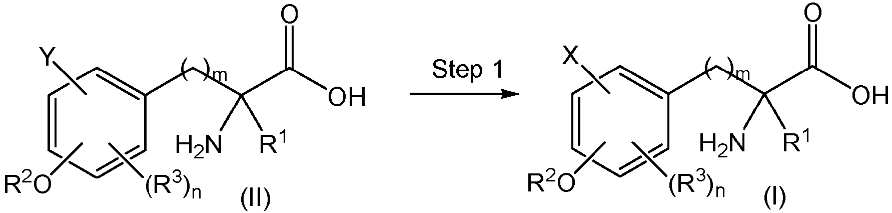 wherein
R¹ is a C₁₋₄ alkyl group;
R² is a C₁₋₆ alkyl group, a C₁₋₆ haloalkyl group or a C₇₋₁₄ aralkyl group;
R³ is a fluorine atom or a chlorine atom;
m is 0, 1 or 2;
n is 0, 1 or 2;
X is a radionuclide selected from ²¹¹At, ²¹⁰At, ¹³¹I, ¹²⁵I, and
⁷⁷Br; and
Y is a boryl group (-B(OH)₂) or its ester group,
Step 1: a step of reacting a compound represented by Formula (II) or a salt thereof with a radionuclide selected from ²¹¹At, ²¹⁰At, ¹³¹I, ¹²⁵I, and ⁷⁷Br in the presence of a reagent selected from alkali metal iodide, alkali metal bromide, N-bromosuccinimide, N-chlorosuccinimide, N-iodosuccinimide and hydrogen peroxide, in water to obtain a radiolabeled compound represented by Formula (I) or a pharmaceutically acceptable salt thereof.

22. A compound represented by Formula (III): 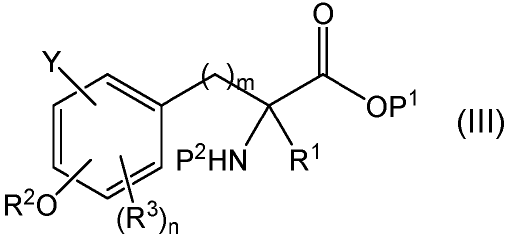 wherein
R¹ is a C₁₋₄ alkyl group;
R² is a C₁₋₆ alkyl group, a C₁₋₆ haloalkyl group or a C₇₋₁₄ aralkyl group;
R³ is a fluorine atom or a chlorine atom;
m is 0, 1 or 2;
n is 0, 1 or 2;
Y is a boryl group (-B(OH)₂) or its ester group;
P¹ is a carboxy-protecting group; and
P² is an amino-protecting group.

23. The compound or a salt thereof according to Claim 22, wherein P¹ is a benzyl group, and P² is a tert-butoxycarbonyl group.

24. A compound represented by Formula (IV'): 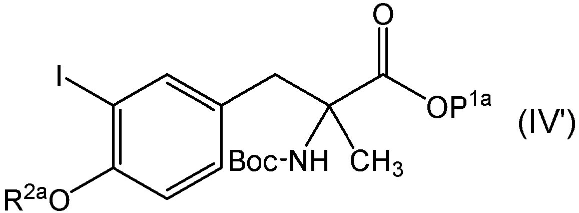 wherein R^{2a} is a C₁₋₃ alkyl group, and P^{1a} is a benzyl group or a C₁₋₃ alkyl group.
